# EUROPEAN PATENT SPECIFICATION

(11) **EP 4 192 557 B1**
(45) Date of publication and mention of the grant of the patent: **25.03.2026**
(21) Application number: 21853648.0
(22) Date of filing: 06.08.2021
(51) Int. Cl.: A61M 16/06, A61B 5/08

(54) **A PATIENT INTERFACE AND A RESPIRATORY SUPPORT SYSTEM**
PATIENTENSCHNITTSTELLE UND ATEMUNTERSTÜTZUNGSSYSTEM
INTERFACE PATIENT ET SYSTÈME D'ASSISTANCE RESPIRATOIRE

(30) Priority: 06.08.2020 US 202063062060 P
(43) Date of publication of application: 14.06.2023
(73) Proprietor: Fisher & Paykel Healthcare Limited, East Tamaki Auckland 2013 (NZ)
(72) Inventor: RUSSELL, David Martin, Auckland, 2013 (NZ); ROBERTSON, Jeremy Patrick, Auckland, 2013 (NZ); SIEW, Silas Sao Jin, Auckland, 2013 (NZ); SALMON, Andrew Paul Maxwell, Auckland, 2013 (NZ); GULLEY, Anton Kim, Auckland, 2013 (NZ); ROWE, Philip Ian, Auckland, 2013 (NZ); BRIDGE, Francis George, Auckland, 2013 (NZ)
(74) Representative: Haslinde, Thorsten
(86) International application number: PCT/NZ2021/050125
(87) International publication number: WO 2022/031178

(56) References cited:
- EP-A1- 2 702 942
- EP-A1- 2 702 942
- EP-A1- 3 626 294
- EP-B1- 2 994 181
- WO-A1-2013/090999
- WO-A1-2013/090999
- WO-A2-2006/072231
- WO-A2-2008/019294
- WO-A2-2008/019294
- WO-A2-2008/076230
- US-A1- 2012 078 069
- US-A1- 2016 279 359
- US-A1- 2019 175 064

## Description

### FIELD OF THE DISCLOSURE

The present disclosure generally relates to patient interfaces and respiratory support systems for providing a breathable gases flow to patients, and more particularly to respiratory support systems with sensors on or near the patient interface.

### BACKGROUND

When providing respiratory support to a patient, it can be beneficial to monitor one or more patient parameters during the course of the therapy. In order to measure these patient parameters one or more patient sensors is used, such as a pulse oximeter, which can be used to determine blood oxygen saturation and heart rate. These parameters can be used individually or in conjunction with further parameters in making an assessment of the patient's health. Additionally, these parameters can be used to adjust one or more control parameters of the respiratory support system being used to provide respiratory support to the patient. This adjustment can be done manually by a clinician, or automatically by a controller of the respiratory support system, such as through feedback control. The parameters being adjusted can include any one or more of flow rate, pressure, temperature, humidity, dew point, oxygen concentration, and/or oxygen saturation.

WO 2008/019294 A2 discloses a patient interface for communicating fluids to and/or from a patient's nasal cavity and/or oral cavity is disclosed. In addition, a patient interface for fluid and physiological function monitoring proximate to the patient's nasal cavity and/or oral cavity is disclosed. An apnea monitor and a method for monitoring apnea are also disclosed.

### SUMMARY

The systems, methods and devices described herein have innovative aspects, no single one of which is indispensable or solely responsible for their desirable attributes. Without limiting the scope of the claims, some of the advantageous features will now be summarized.

Throughout, the term `respiratory support system' in this specification means the combination of a respiratory support apparatus and any associated components used for providing respiratory support to a patient, such as a patient interface and/or one or more gases conduits and/or other components used to provide respiratory support to a patient. Components that may make up at least a part of the respiratory support apparatus can include any one or more of: a flow generator, a controller, a humidifier, a graphical user interface, a flow control valve.

Throughout, the term 'circuit' in this specification means the entire breathable gases inspiratory pathway to the patient from a gases supply, and may also include the expiratory gases path away from the patient to a gases supply. The circuit at a minimum therefore includes the inspiratory gases pathway (including all the components) from the gases supply to the patient interface. The interface itself e.g. mask or cannula, is separate from the gases pathway and not part of the 'circuit'.

Throughout this specification, a 'gases conduit' is any passageway configured to transport a breathable gases flow.

Throughout this specification, the terms 'clinician', 'patient', and 'user' may be used to refer to individuals who may interact with the respiratory support apparatus. The term 'patient' as used in this specification means the individual who is receiving therapy (e.g. a therapeutic gases flow) from the respiratory support system, and in particular is the individual who is wearing the patient interface. The term 'clinician' as used in this specification means an individual such as a nurse or a doctor who is not receiving therapy from the respiratory support system, but might adjust the settings of the respiratory support apparatus, aid in setting up the respiratory support system, and/or help to affix the patient interface to the patient, or prescribe therapy, among other tasks. The term 'user' as used in this specification means an individual who may adjust the settings of the respiratory support apparatus, aid in setting up the respiratory support system, and/or help to affix the patient interface to the patient, among other tasks. Depending on the situation, the user may be a clinician or the patient themselves. For example, in a hospital scenario, a clinician is likely setup the system for the patient, and as such the term 'user' likely refers to the clinician. For scenarios in which the 'user' is adjusting the operating parameters of the respiratory support apparatus but not necessarily interacting with the patient, the user may be a biomedical engineer or a maintenance engineer or a technician. Conversely, in a scenario in which the patient is using the respiratory support system at home, the patient may set the system themselves, and as such the term 'user' likely refers to the patient.

According to the present invention there is provided a nasal cannula according to claim 1.

According to an aspect of this disclosure there is provided a patient interface for supplying a gases flow to a patient comprising:
a body configured to engage with an orifice of the patient and direct the gases flow to said orifice, and
one or more sensors configured to measure a parameter,
wherein the one or more sensors are mounted (i.e. positioned) on any one or more of:
   a) the patient interface;
   b) headgear configured to mount the patient interface on the patient's head;
   c) a headgear connector configured to connect the headgear to the patient interface;
   d) a gas delivery conduit configured to deliver breathable gases to the patient.

According to an aspect of this disclosure there is provided a patient interface for supplying a gases flow to a patient comprising:
a body configured to engage with an orifice of the patient and direct the gases flow to said orifice, and
a frame on which the body is permanently or removably mounted, the frame configured to connect to headgear to mount the patient interface on the patient's head;
one or more sensors configured to measure a parameter,
wherein the one or more sensors are mounted on the patient interface and/or the frame and/or the headgear.

According to an aspect of this disclosure there is provided a patient interface for supplying a gases flow to a patient comprising:
a body configured to engage with an orifice of the patient and direct the gases flow to said orifice, and
lateral arms extending laterally outwardly from the body and configured to connect to headgear to mount the patient interface on the patient's head;
one or more sensors configured to measure a parameter,
wherein the one or more sensors are mounted on the patient interface and/or the lateral arms and/or the headgear.

The one or more sensors may be removably mounted (i.e. can be removed from the patient interface and re-positioned) into any one or more of:
a) the patient interface;
b) headgear configured to mount the patient interface on the patient's head;
c) a headgear connector configured to connect the headgear to the patient interface;
d) a gas delivery conduit configured to deliver breathable gases to the patient.

The one or more sensors being removable can be advantageous because the sensors can be removed, cleaned and mounted into another interface. This allows the sensors to be re-used between different patients.

Alternatively the one or more sensors may be integrated into one or more of:
a) the patient interface;
b) headgear configured to mount the patient interface on the patient's head;
c) a headgear connector configured to connect the headgear to the patient interface;
d) a gas delivery conduit configured to deliver breathable gases to the patient.

The one or more sensors may be integrated such that they cannot be removed. In this integrated arrangement the one or more sensors may be disposable.

According to an aspect of this disclosure there is provided a patient interface for supplying a gases flow to a patient comprising:
a body configured to engage with an orifice of the patient and direct the gases flow to said orifice, and
one or more sensors configured to measure a parameter,
wherein the body further comprises a top surface and a rear surface, the rear surface being adjacent the patient in use of the patient interface; wherein
an outer surface of the one or more sensors is flush with the top surface or the rear surface.

According to an aspect of this disclosure there is provided a patient interface for supplying a gases flow to a patient comprising:
a body configured to engage with an orifice of the patient and direct the gases flow to said orifice, and
one or more sensors configured to measure a parameter,
wherein the one or more sensors are embedded below an outer surface of the body of the patient interface.

At least one of the one or more sensors may be a patient sensor, and the parameter may be a physiological parameter of the patient.

The parameter may be a measure of blood oxygenation of the patient.

The patient interface may sealingly engage with the orifice of the patient.

The patient interface may comprise a mask.

The mask may be a nasal mask.

The mask may be an oral mask.

The mask may be a nasal mask.

The mask may be a full face mask.

The mask may comprise a cushion.

The one or more sensors may be flush with an outer surface of the cushion.

The patient interface may comprise a nasal pillows interface.

The patient interface may comprise a tracheostomy interface.

The patient interface may further comprise a head securement assembly.

The head securement assembly may comprise one or more straps.

The head securement assembly may comprise one or more facial pads.

The or each facial pad may comprise an adhesive surface to adhere to the patient's skin.

The or each facial pad may comprise two distinct patches.

The two distinct patches may be removably coupled.

The one or more sensors may be a pulse oximeter.

The pulse oximeter may be a reflectance type pulse oximeter.

The one or more sensors may be arranged to contact the patient's columella whilst the patient interface is in use.

The patient interface may further comprise:
a gases inlet conduit for receiving the gases flow from a flow source, the gases inlet conduit defining at least a portion of a gases flow path; and
an interface connector for receiving the gases flow from the gases inlet conduit and directing the gases flow towards the patient.

The patient interface may further comprise a set of wires, wherein the gases inlet conduit further comprises a patient end, and a distal end, and
wherein the patient end is connected to the interface connector,
the distal end comprises an interface inlet, the interface inlet comprising a set of electrical contacts, and
the set of wires of the patient interface provides electrical communication between the one or more sensors and the set of electrical contacts of the interface inlet.

The set of electrical contacts of the interface inlet may comprise a planar surface, and the planar surface may be substantially perpendicular to a longitudinal axis of a lumen of the interface inlet.

The set of electrical contacts of the interface inlet may comprise a pin and/or a socket of a pin and socket electrical connector, and a longitudinal axis of the pin and/or socket may be substantially parallel to a longitudinal axis of a lumen of the interface inlet.

The set of electrical contacts of the interface inlet may be in a fixed position relative to the remainder of the interface inlet.

The patient interface may further comprise a mesh layer surrounding the outer surface of at least a portion of the body of the patient interface or the gases inlet conduit, wherein the mesh may comprise a plurality of interwoven filaments, and at least a portion of the set of wires of the patient interface may be interwoven with the filaments of the mesh layer.

At least a portion of the set of wires of the patient interface may be embedded into at least a portion of the body of the patient interface, the interface connector of the patient interface, or the gases inlet conduit.

At least a portion of the set of wires of the patient interface may be located on an outer surface of at least a portion of the body of the patient interface, the interface connector of the patient interface, or the gases inlet conduit.

At least a portion of the set of wires of the patient interface may be located on an inner surface of at least a portion of the body of the patient interface, the interface connector of the patient interface, or the gases inlet conduit.

According to an aspect of this disclosure there is provided a respiratory support system for generating a gases flow comprising:
a respiratory support apparatus comprising:
   a flow generator,
   a respiratory support apparatus outlet, and
   a controller,
an inspiratory conduit comprising:
   a patient end having an inspiratory conduit outlet, and
   an apparatus end having an inspiratory conduit inlet,
the patient interface of any of the preceding or subsequent statements;
wherein the respiratory support apparatus outlet is configured to form a pneumatic and electrical connection with the inspiratory conduit inlet,
wherein the respiratory support apparatus outlet is in electrical communication with the controller, and
the controller is configured to supply power to and receive data from the one or more sensors.

The flow generator may be a blower.

The respiratory support system may further comprise a humidifier for adding heat and/or humidity to the gases flow.

The respiratory support system may further comprise an ambient air inlet.

The respiratory support system may further comprise at least one supplemental gas inlet for receiving a flow of supplemental gases.

The respiratory support system may further comprise a valve to regulate the flow of supplemental gases through the at least one supplemental gas inlets.

The valve may be a proportional valve.

The at least one supplemental gas inlet may be an oxygen inlet.

The respiratory support apparatus may comprise at least one gases composition sensor to measure the composition of the gases flow.

The at least one gases composition sensor may comprise an ultrasonic sensor system.

The controller may be configured to:
receive a measure of the composition of the gases flow from the at least one gases composition sensor;
compare the measure of the composition of the gases flow with a target gases composition; and
adjust the position of the valve based at least in part on the comparison between the two values.

The target gases composition may be set by a user.

The controller may be configured to:
receive a measure of the parameter from the one or more sensors;
compare the measure of the parameter with a target value for the parameter; and
adjust the target gases composition based at least in part on the comparison between the measure and the target value.

The target parameter may be set by a user.

The measure of the parameter may be used by the controller to determine when the patient is using the patient interface.

According to an aspect of this disclosure there is provided a patient interface for supplying a gases flow to a patient comprising:
one or more sensors configured to be placed on the patient's skin and configured to measure at least one parameter, and
a body configured to engage an orifice of the patient and direct the gases flow to said orifice,
wherein the one or more sensors are moveable relative to the body of the patient interface.

The patient interface may further comprise a gases inlet conduit for receiving the gases flow from a flow source, the gases inlet conduit defining at least a portion of a gases flow path and comprising a patient end and a distal end.

The patient interface may further comprise a first set of wires.

The patient interface may further comprise an interface connector for receiving the gases flow from the gases inlet conduit and directing the gases flow towards the patient.

The patient end may be connected to the interface connector.

The distal end may comprise an interface inlet, the interface inlet comprising a set of electrical contacts.

The first set of wires of the patient interface may provide electrical communication between the one or more sensors and the set of electrical contacts of the interface inlet.

According to an aspect of this disclosure there is provided a patient interface for supplying a gases flow to a patient comprising:
a gases inlet conduit for receiving the gases flow from a flow source, the gases inlet conduit defining at least a portion of a gases flow path and comprising a patient end and a distal end,
a first set of wires,
one or more sensors configured to be placed on the patient's skin and configured to measure at least one parameter,
a body, and
an interface connector for receiving the gases flow from the gases inlet conduit, and wherein the patient end is connected to the interface connector,
the distal end comprises an interface inlet, the interface inlet comprising a set of electrical contacts, and
the first set of wires of the patient interface provides electrical communication between the one or more sensors and the set of electrical contacts of the interface inlet.

The patient interface may sealingly engage with the orifice of the patient.

The patient interface may comprise a mask.

The mask may be a nasal mask.

The mask may be an oral mask.

The mask may be a nasal mask.

The mask may be a full face mask.

The mask may comprise a cushion.

The one or more sensors may be flush with an outer surface of the cushion.

The patient interface may comprise a nasal pillows interface.

The patient interface may comprise a tracheostomy interface.

At least one of the one or more sensors may be a patient sensor, and the parameter may be a physiological parameter of the patient.

The physiological parameter may be a measure of blood oxygenation of the patient.

At least one of the one or more sensors may be a pulse oximeter.

The pulse oximeter may be a reflectance type pulse oximeter.

The pulse oximeter may be a transmissive pulse oximeter.

The patient interface may further comprise a head securement assembly.

The head securement assembly comprise one or more straps.

The sensors may be moveable relative to the body of the patient interface.

The patient interface may further comprise a sensor arm, wherein the one or more sensors may be located on the sensor arm.

The sensor arm may be rigid such that it cannot be easily bent by a user.

The sensor arm may be deformable such that it can be easily bent by a user. The sensor arm may be non-elastically deformable such that the sensor arm may remain in a bent condition, once deformed by the user. The sensor arm may be resiliently deformable.

A surface of the sensor arm may comprise an adhesive such that the surface is capable of adhering to the patient's skin.

A length of the sensor arm may be adjustable.

The length of sensor arm may be adjustable through telescopic motion.

The head securement assembly may further comprise a sensor mount connected to one of the straps, wherein the sensor arm projects from the sensor mount.

The sensor mount may be moveably connected to one of the straps.

The sensor mount may be slidably connected to one of the straps.

The sensor mount may be removably connected to one of the straps.

The patient interface may further comprise a sensor mount connected to one the gases inlet conduit, wherein the sensor arm projects from the sensor mount.

The sensor mount may be moveably connected to the gases inlet conduit.

The sensor mount may be slidably connected to the gases inlet conduit.

The sensor mount may be removably connected to the gases inlet conduit.

The sensor arm may be moveable relative to the sensor mount.

The sensor arm may be slidably mounted to the sensor mount.

The sensor arm may be slidably mounted to the sensor mount so as to be able to slide in a direction parallel to the length of the strap or gases inlet conduit to which the sensor mount is connected.

The sensor arm may be slidably mounted to the sensor mount so as to be able to slide in a direction transverse to the length of the strap or gases inlet conduit which the sensor mount is connected to.

The sensor arm may be configured to rotate about an axis at which it connects to the sensor mount.

The patient interface may further comprise a sensor clip configured to clip onto the patient, wherein the one or more sensors may be located on the sensor clip.

The sensor clip may be configured to clip onto the patient's ear.

The gases inlet conduit may be substantially rigid.

The gases inlet conduit may be substantially flexible.

The gases inlet conduit may be integrally formed with the patient interface.

The gases inlet conduit may be releasably connected to the patient interface.

The set of electrical contacts of the interface inlet may comprise a planar surface, and
the planar surface is substantially perpendicular to a longitudinal axis of a lumen of the interface inlet.

The set of electrical contacts of the interface inlet may comprise a pin and/or a socket of a pin and socket electrical connector, and
a longitudinal axis of the pin and/or socket may be substantially parallel to a longitudinal axis of a lumen of the interface inlet.

The set of electrical contacts of the interface inlet may be in fixed position relative to the remainder of the interface inlet.

The patient interface may further comprise a mesh layer surrounding the outer surface of at least a portion of the patient interface or the gases inlet conduit,
wherein the mesh may comprise a plurality of interwoven filaments, and
at least a portion of the first set of wires of the patient interface may be interwoven with the filaments of the mesh layer.

At least a portion of the first set of wires of the patient interface may be embedded into at least a portion of the body of the patient interface, the interface connector of the patient interface, or the gases inlet conduit.

At least a portion of the first set of wires of the patient interface may be located on an outer surface of at least a portion of the body of the patient interface, the interface connector of the patient interface, or the gases inlet conduit.

At least a portion of the first set of wires of the patient interface may be located on an inner surface of at least a portion of the body of the patient interface, the interface connector of the patient interface, or the gases inlet conduit.

The patient interface may further comprise:
a wire coil, and
a second set of wires extending from the sensor to the wire coil, wherein
the second set of wires can be retracted into the wire coil,
the wire coil is connected to the first set of wires, and
at least one of the one or more sensors or the sensor is located at the end of the second set of wires.

The second set of wires may retract into the wire coil automatically.

The second set of wires may retract into the wire coil upon user actuation of a button, switch, or lever.

The wire coil may be mounted to one of the straps.

The wire coil may be removably mounted to one of the straps.

The wire coil may be mounted to the gas inlet conduit.

The wire coil may be removably mounted to the gas inlet conduit.

According to an aspect of this disclosure there is provided a respiratory support system for generating a gases flow comprising:
a respiratory support apparatus comprising
   a flow generator,
   a respiratory support apparatus outlet, and
   a controller,
an inspiratory conduit comprising
   a patient end having an inspiratory conduit outlet, and
   an apparatus end having an inspiratory conduit inlet,
the patient interface of any of the preceding statements, and
wherein the respiratory support apparatus outlet is configured to form a pneumatic and electrical connection with the inspiratory conduit inlet,
wherein the respiratory support apparatus outlet is in electrical communication with the controller, and
the controller is configured to supply power to and receive data from the one or more sensors.

The flow generator may be a blower.

The respiratory support system may further comprise a humidifier for adding heat and/or humidity to the gases flow.

The respiratory support system may further comprise an ambient air inlet.

The respiratory support system may further comprise one or more supplemental gases inlets for receiving a flow of supplemental gases.

At least one of the one or more supplemental gases inlets may be an oxygen inlet.

The respiratory support system may further comprise a valve to regulate the flow of supplemental gases through the at least one of the one or more supplemental gases inlets.

The valve may be a proportional valve.

The respiratory support apparatus may comprise one or more gases composition sensors to measure the composition of the gases flow.

The one or more gases composition sensors may comprise an ultrasonic sensor system.

The controller may be configured to:
receive a measure of the composition of the gases flow from the one or more gases composition sensors,
compare the measure of the composition of the gases flow with a target composition, and
adjust the position of the valve based at least in part on the difference between the two values.

The target gases composition may be set by a user.

The controller may be configured to:
receive a measure of the parameter from the one or more sensors,
compare the measure of the parameter with a target value for the parameter, and
adjust the target gases composition based at least in part on the difference between the measure and target value.

The target value for the parameter may be set by a user.

The measure of the parameter may be used by the controller to determine when the patient is using the patient interface.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the nose of the patient when the patient interface is in use.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the upper lip of the patient when the patient interface is in use.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the lower lip of the patient when the patient interface is in use.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the mouth of the patient when the patient interface is in use.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the cheek of the patient when the patient interface is in use.

According to an aspect of this disclosure there is provided a patient interface for supplying a gases flow to a patient comprising:
a patient interface defining at least a portion of a gases flow path and comprising:
a body configured to engage with an orifice of the patient and direct the gases flow to said orifice, and
one or more sensors configured to measure a parameter,
wherein the sensor is configured to contact the neck of the patient when the patient interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the body further comprises a top surface and a rear surface, the rear surface being adjacent the patient in use of the nasal cannula interface; wherein an outer surface of the one or more sensors is flush with the top surface or the rear surface.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the one or more sensors are embedded below an outer surface of the body of the nasal cannula.

At least one of the one or more sensors may be a patient sensor, and the parameter may be a physiological parameter of the patient.

The parameter may be a measure of blood oxygenation of the patient.

The at least one prong may be configured to be received in one or more nares of the patient.

One or more of the at least one prong may be configured to form a seal with one of the nares of the patient.

One or more of the at least one prong may be configured be received in one of the nares of the patient in an unsealed manner.

The nasal cannula may further comprise a head securement assembly.

The head securement assembly may comprise one or more straps.

The head securement assembly may comprise one or more facial pads.

The or each facial pad may comprise an adhesive surface to adhere to the patient's skin.

The or each facial pad may comprise two distinct patches.

The two distinct patches may be removably coupled.

The nasal cannula may further comprise a pair of side arms.

The pair of side arms may be integral with the body of the nasal cannula.

The head securement assembly may be connected to the side arms.

The facial pads may be located on the side arms.

The outer surface of the patient sensor may be flush with the top surface, and the top surface may be a patient contacting surface, or the outer surface of the patient sensor may be flush with the rear surface and the rear surface may be a patient contacting surface.

The one or more sensors may be a pulse oximeter.

The pulse oximeter may be a reflectance type pulse oximeter.

The nasal cannula may further comprise a second prong extending from the base portion.

The one or more sensors may be located between the two prongs.

The at least one prong may extend from the top surface of the body of the nasal cannula, and the one or more sensors may be located on said top surface.

The one or more sensors may be arranged to contact the patient's columella whilst the nasal cannula interface is in use.

The at least one prong may extend from a top surface of the body of the nasal cannula, and the one or more sensors may be located on a surface of the body that is adjacent to said top surface.

The one or more sensors may be positioned on the body of the nasal cannula so as to contact the patient's upper lip whilst the nasal cannula interface is in use.

The nasal cannula interface may further comprise:
a gases inlet conduit for receiving the gases flow from a flow source, the gases inlet conduit defining at least a portion of a gases flow path; and
an interface connector for receiving the gases flow from the gases inlet conduit and directing the gases flow towards the at least one prong.

The nasal cannula interface may further comprise a set of wires, wherein the gases inlet conduit further comprises a patient end, and a distal end, and
wherein the patient end is connected to the interface connector,
the distal end comprises an interface inlet, the interface inlet comprising a set of electrical contacts, and
the set of wires of the nasal cannula interface provides electrical communication between the one or more sensors and the set of electrical contacts of the interface inlet.

The set of electrical contacts of the interface inlet may comprise a planar surface, and the planar surface may be substantially perpendicular to a longitudinal axis of a lumen of the interface inlet.

The set of electrical contacts of the interface inlet may comprise a pin and/or a socket of a pin and socket electrical connector, and a longitudinal axis of the pin and/or socket may be substantially parallel to a longitudinal axis of a lumen of the interface inlet.

The set of electrical contacts of the interface inlet may be in a fixed position relative to the remainder of the interface inlet.

The nasal cannula interface may further comprise a mesh layer surrounding the outer surface of at least a portion of the nasal cannula or the gases inlet conduit,
wherein the mesh may comprise a plurality of interwoven filaments, and
at least a portion of the set of wires of the nasal cannula interface may be interwoven with the filaments of the mesh layer.

At least a portion of the set of wires of the nasal cannula interface may be embedded into at least a portion of the body of the nasal cannula, the interface connector of the nasal cannula, or the gases inlet conduit.

At least a portion of the set of wires of the nasal cannula interface may be located on an outer surface of at least a portion of the body of the nasal cannula, the interface connector of the nasal cannula, or the gases inlet conduit.

At least a portion of the set of wires of the nasal cannula interface may be located on an inner surface of at least a portion of the body of the nasal cannula, the interface connector of the nasal cannula, or the gases inlet conduit.

According to an aspect of this disclosure there is provided a respiratory support system for generating a gases flow comprising:
a respiratory support apparatus comprising:
   a flow generator,
   a respiratory support apparatus outlet, and
   a controller,
an inspiratory conduit comprising:
   a patient end having an inspiratory conduit outlet, and
   an apparatus end having an inspiratory conduit inlet,
the nasal cannula interface of any of the preceding statements;
wherein the respiratory support apparatus outlet is configured to form a pneumatic and electrical connection with the inspiratory conduit inlet,
wherein the respiratory support apparatus outlet is in electrical communication with the controller, and
the controller is configured to supply power to and receive data from the one or more sensors.

The flow generator may be a blower.

The respiratory support system may further comprise a humidifier for adding heat and/or humidity to the gases flow.

The respiratory support system may further comprise an ambient air inlet.

The respiratory support system may further comprise at least one supplemental gas inlet for receiving a flow of supplemental gases.

The respiratory support system may further comprise a valve to regulate the flow of supplemental gases through the at least one supplemental gas inlets.

The valve may be a proportional valve.

The at least one supplemental gas inlet may be an oxygen inlet.

The respiratory support apparatus may comprise at least one gases composition sensor to measure the composition of the gases flow.

The at least one gases composition sensor may comprise an ultrasonic sensor system.

The controller may be configured to:
receive a measure of the composition of the gases flow from the at least one gases composition sensor,
compare the measure of the composition of the gases flow with a target gases composition, and
adjust the position of the valve based at least in part on the comparison between the two values.

The target gases composition may be set by a user.

The controller may be configured to:
receive a measure of the parameter from the one or more sensors,
compare the measure of the parameter with a target value for the parameter, and
adjust the target gases composition based at least in part on the comparison between the measure and the target value.

The target parameter may be set by a user.

The measure of the parameter may be used by the controller to determine when the patient is using the nasal cannula interface.

According to an aspect of this disclosure there is provided a nasal cannula interface for supplying a gases flow to a patient comprising:
one or more sensors configured to be placed on the patient's skin and configured to measure at least one parameter, and
a nasal cannula defining at least a portion of a gases flow path and comprising
   a body having a base portion and at least one prong extending from the base portion, the at least one prong being configured to direct the gases flow to an orifice of the patient,
wherein the one or more sensors are moveable relative to the body of the nasal cannula.

The nasal cannula interface may further comprise a gases inlet conduit for receiving the gases flow from a flow source, the gases inlet conduit defining at least a portion of a gases flow path and comprising a patient end and a distal end.

The nasal cannula interface may further comprise a first set of wires.

The nasal cannula may further comprise an interface connector for receiving the gases flow from the gases inlet conduit and directing the gases flow towards the at least one prong.

The patient end may be connected to the interface connector.

The distal end may comprise an interface inlet, the interface inlet comprising a set of electrical contacts.

The first set of wires of the nasal cannula interface may provide electrical communication between the one or more sensors and the set of electrical contacts of the interface inlet.

According to an aspect of this disclosure there is provided a nasal cannula interface for supplying a gases flow to a patient comprising:
a gases inlet conduit for receiving the gases flow from a flow source, the gases inlet conduit defining at least a portion of a gases flow path and comprising a patient end and a distal end,
a first set of wires,
one or more sensors configured to be placed on the patient's skin and configured to measure at least one parameter, and
a nasal cannula defining at least a portion of a gases flow path and comprising
   a body having a base portion and at least one prong extending from the base portion, the at least one prong being configured to direct the gases flow to an orifice of the patient, and
   an interface connector for receiving the gases flow from the gases inlet conduit and directing the gases flow towards the at least one prong, and
wherein the patient end is connected to the interface connector,
the distal end comprises an interface inlet, the interface inlet comprising a set of electrical contacts, and
the first set of wires of the nasal cannula interface provides electrical communication between the one or more sensors and the set of electrical contacts of the interface inlet.

The at least one prong may be configured to be received in one or more nares of the patient.

The at least one prong may be configured to form a seal with one of the nares of the patient.

The at least one prong may be configured to be received in one of the nares of the patient in an unsealed manner.

The nasal cannula interface may further comprise a second prong extending from the base portion.

At least one of the one or more sensors may be a patient sensor, and the parameter may be a physiological parameter of the patient.

The physiological parameter may be a measure of blood oxygenation of the patient.

At least one of the one or more sensors may be a pulse oximeter.

The pulse oximeter may be a reflectance type pulse oximeter.

The pulse oximeter may be a transmissive pulse oximeter.

The body of the nasal cannula further comprise a pair of side arms.

The nasal cannula interface may further comprise a head securement assembly.

The head securement assembly may be connected to the side arms.

The head securement assembly comprise one or more straps.

The sensors may be moveable relative to the body of the nasal cannula interface.

The nasal cannula interface may further comprise a sensor arm, wherein the one or more sensors may be located on the sensor arm.

The sensor arm may be rigid such that it cannot be easily bent by a user.

The sensor arm may be resiliently deformable such that it can be easily bent by a user.

A surface of the sensor arm may comprise an adhesive such that the surface is capable of adhering to the patient's skin.

A length of the sensor arm may be adjustable.

The length of sensor arm may be adjustable through telescopic motion.

The head securement assembly may further comprise a sensor mount connected to one of the straps, wherein the sensor arm projects from the sensor mount.

The sensor mount may be moveably connected to one of the straps.

The sensor mount may be slidably connected to one of the straps.

The sensor mount may be removably connected to one of the straps.

The nasal cannula interface may further comprise a sensor mount connected to one the gases inlet conduit, wherein the sensor arm projects from the sensor mount.

The sensor mount may be moveably connected to the gases inlet conduit.

The sensor mount may be slidably connected to the gases inlet conduit.

The sensor mount may be removably connected to the gases inlet conduit.

The sensor arm may be moveable relative to the sensor mount.

The sensor arm may be slidably mounted to the sensor mount.

The sensor arm may be slidably mounted to the sensor mount so as to be able to slide in a direction parallel to the length of the strap or gases inlet conduit to which the sensor mount is connected.

The sensor arm may be slidably mounted to the sensor mount so as to be able to slide in a direction transverse to the length of the strap or gases inlet conduit which the sensor mount is connected to.

The sensor arm may be configured to rotate about an axis at which it connects to the sensor mount.

The nasal cannula interface may further comprise a sensor clip configured to clip onto the patient, wherein the one or more sensors may be located on the sensor clip.

The sensor clip may be configured to clip onto the patient's ear.

The gases inlet conduit may be substantially rigid.

The gases inlet conduit may be substantially flexible.

The gases inlet conduit may be integrally formed with the nasal cannula.

The gases inlet conduit may be releasably connected to the nasal cannula.

The set of electrical contacts of the interface inlet may comprise a planar surface, and
the planar surface is substantially perpendicular to a longitudinal axis of a lumen of the interface inlet.

The set of electrical contacts of the interface inlet may comprise a pin and/or a socket of a pin and socket electrical connector, and
a longitudinal axis of the pin and/or socket may be substantially parallel to a longitudinal axis of a lumen of the interface inlet.

The set of electrical contacts of the interface inlet may be in fixed position relative to the remainder of the interface inlet.

The nasal cannula interface may further comprise a mesh layer surrounding the outer surface of at least a portion of the nasal cannula or the gases inlet conduit,
wherein the mesh may comprise a plurality of interwoven filaments, and
at least a portion of the first set of wires of the nasal cannula interface may be interwoven with the filaments of the mesh layer.

At least a portion of the first set of wires of the nasal cannula interface may be embedded into at least a portion of the body of the nasal cannula, the interface connector of the nasal cannula, or the gases inlet conduit.

At least a portion of the first set of wires of the nasal cannula interface may be located on an outer surface of at least a portion of the body of the nasal cannula, the interface connector of the nasal cannula, or the gases inlet conduit.

At least a portion of the first set of wires of the nasal cannula interface may be located on an inner surface of at least a portion of the body of the nasal cannula, the interface connector of the nasal cannula, or the gases inlet conduit.

The nasal cannula interface may further comprise:
a wire coil, and
a second set of wires extending from the sensor to the wire coil, wherein
the second set of wires can be retracted into the wire coil,
the wire coil is connected to the first set of wires, and
at least one of the one or more sensors or the sensor is located at the end of the second set of wires.

The second set of wires may retract into the wire coil automatically.

The second set of wires may retract into the wire coil upon user actuation of a button, switch, or lever.

The wire coil may be mounted to one of the straps.

The wire coil may be removably mounted to one of the straps.

The wire coil may be mounted to the gas inlet conduit.

The wire coil may be removably mounted to the gas inlet conduit.

The nasal cannula interface may be configured such that the one or more sensor(s) are connected to one or more sensor wires via an inductive coupling. For example, the one or more sensor(s) may be configured to be connected to one or more sensor wires in a gases delivery conduit via the inductive coupling. Such a coupling can avoid the need for a physical electrical connector and exposed electrical contacts.

According to another aspect of this disclosure there is provided headgear for a patient interface comprising
a strap forming a part of the headgear for assisting in retaining or stabilising of a patient interface upon a user,
a first connector at a first end portion of the strap for connecting the strap to the patient interface, and
a first cheek engaging member adapted to encapsulate the first connector and having a surface region adapted to locate between the user's cheek and the connector to minimise direct contact of the connector with the user's skin in use;
wherein one or more sensors are configured to be placed on or adjacent the patient's skin and configured to measure at least one parameter; the one or more sensors being mounted on the first cheek engaging member.

The headgear may further comprise a second connector at a second opposing end portion of the strap for connecting the strap to the patient interface, and a second cheek engaging member configured to encapsulate the second connector and having a surface region adapted to locate between the user's other cheek to minimise direct contact of the connector with the user's skin in use.

Each cheek engaging member may be configured to removably couple about the respective connector.

The surface region of each cheek engaging member may comprise a material that is substantially softer than a material of the respective connector.

The surface region of each cheek engaging member may comprise a relatively higher frictional surface material than the respective connector, to assist with retaining or stabilising of a patient interface upon the face of a user.

The material may be a thermoplastic elastomer.

The surface region of each cheek engaging member may be a surface of wider surface area at an end of the respective cheek engaging member more adjacent to the patient interface than a surface area of an opposing end of the cheek member more distant from the patient interface.

The surface region of each cheek engaging members may taper from a relatively wider end to a relatively lesser end.

Each cheek engaging member may be a sleeve configured to receivably retain the respective connector therein.

The sleeve may be configured to removably couple about the respective connector.

The connector may be adapted to extend through a passage in the sleeve.

The sensor may be connected to one or more sensor wires, the one or more sensor wires extending through the passage in the sleeve.

Each connector may be substantially housed by the respective sleeve in a region adapted to locate adjacent the user's cheek in use.

Each sleeve may be curved along at least a portion of the length of the sleeve to complement the contour of the respective cheek.

Each connector may be curved along at least a portion of the length of the connector adapted to locate adjacent the respective cheek.

The connector may be pre-formed with a curved profile.

Each sleeve may be pre-formed with a curved profile.

Each sleeve may be curved upon encapsulating the respective connector.

Each connector comprises a clip for releasably connecting with the patient interface.

Each connector may be frictionally or mechanically engaged with the respective cheek engaging member once in-situ.

The one or more sensor(s) may be mounted on the sleeve.

The one or more sensor(s) may be removably mounted on the cheek member.

An outer surface of the one or more sensors may be flush with the surface region of the cheek member.

According to an aspect of this disclosure there is provided a respiratory support system for generating a gases flow comprising:
a respiratory support apparatus comprising
   a flow generator,
   a respiratory support apparatus outlet, and
   a controller,
an inspiratory conduit comprising
   a patient end having an inspiratory conduit outlet, and
   an apparatus end having an inspiratory conduit inlet,
the nasal cannula interface or headgear of any of the preceding statements, and
wherein the respiratory support apparatus outlet is configured to form a pneumatic and electrical connection with the inspiratory conduit inlet,
wherein the respiratory support apparatus outlet is in electrical communication with the controller, and
the controller is configured to supply power to and receive data from the one or more sensors.

The flow generator may be a blower.

The respiratory support system may further comprise a humidifier for adding heat and/or humidity to the gases flow.

The respiratory support system may further comprise an ambient air inlet.

The respiratory support system may further comprise one or more supplemental gases inlets for receiving a flow of supplemental gases.

At least one of the one or more supplemental gases inlets may be an oxygen inlet.

The respiratory support system may further comprise a valve to regulate the flow of supplemental gases through the at least one of the one or more supplemental gases inlets.

The valve may be a proportional valve.

The respiratory support apparatus may comprise one or more gases composition sensors to measure the composition of the gases flow.

The one or more gases composition sensors may comprise an ultrasonic sensor system.

The controller may be configured to:
receive a measure of the composition of the gases flow from the one or more gases composition sensors;
compare the measure of the composition of the gases flow with a target composition; and
adjust the position of the valve based at least in part on the difference between the two values.

The target gases composition may be set by a user.

The controller may be configured to:
receive a measure of the parameter from the one or more sensors;
compare the measure of the parameter with a target value for the parameter; and
adjust the target gases composition based at least in part on the difference between the measure and target value.

The target value for the parameter may be set by a user.

The measure of the parameter may be used by the controller to determine when the patient is using the nasal cannula interface.

According to an aspect of this disclosure there is provided a nasal cannula interface for supplying a gases flow to a patient comprising:
a nasal cannula defining at least a portion of a gases flow path and comprising a body having a base portion and at least one prong extending from the base portion, the at least one prong being configured to direct the gases flow to an orifice of the patient, the body comprising a lateral mount; and
headgear according to any one of the above statements; wherein the first connector of the headgear is connected to the lateral mount of the body.

The body may comprise a recess positioned between a pair of prongs, wherein the prongs extend from the body. The sensor may be located in the recess, between the prongs.

The nasal cannula interface may comprise a manifold part received within an opening in the body (i.e. face mount part), wherein the manifold part comprises a dip, wherein the dip in the manifold portion aligns with the recessed in the body when the manifold is inserted into the body .

According to an aspect of this disclosure there is provided a nasal cannula interface for supplying a gases flow to a patient comprising a nasal cannula defining at least a portion of a gases flow path and comprising:
a body having a base portion and pair of prongs extending from the base portion, the prongs being configured to direct the gases flow to an orifice of the patient;
a gases flow manifold part comprising a gases inlet for receiving a flow of gas from a gas source, and a gases outlet for delivering the flow of gas to the prongs of the body;
the nasal cannula interface comprising a patient sensor configured to measure a parameter; the patient sensor being positioned between the prongs, the body comprising a recess adjacent the face of the patient.

The patient sensor may comprise a pulse oximeter.

The nasal cannula interface may comprise a plurality of patient sensors.

The gases flow manifold part may comprise a recessed portion between the prongs and the sensor positioned in the recess, and
the nasal cannula further comprising a manifold part received within an opening in the body (i.e. face mount part), wherein the manifold part comprises a dip, wherein the dip in the manifold portion aligns with the recessed in the body when the manifold is inserted into the body.

According to an aspect of this disclosure there is provided a nasal cannula interface comprising: a face mount part having a base portion and at least one nasal prong extending from the base portion and capable of fitting in at least one of a user's nares, and
a gases flow manifold part having a gases inlet for receiving a flow of gas from a gas source, and a gases outlet for delivering the flow of gas to the at least one nasal prong of the face mount part, the manifold part being adapted to be received by the base portion of the face mount part to fluidly connect the outlet of the manifold with the at least one nasal prong of the face mount part, and wherein the manifold part further comprises a recess; a patient sensor being positioned in the recess.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient; and
one or more sensors configured to measure a parameter;
wherein the sensor is configured to contact the nose of the patient when the nasal cannula interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the sensor is configured to contact the upper lip of the patient when the nasal cannula interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the sensor is configured to contact the lower lip of the patient when the nasal cannula interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the sensor is configured to contact the mouth of the patient when the nasal cannula interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the sensor is configured to contact the cheek of the patient when the nasal cannula interface is in use.

According to an aspect of this disclosure there is provided a nasal cannula interface for supplying a gases flow to a patient comprising:
a nasal cannula defining at least a portion of a gases flow path and comprising:
a body having a base portion and at least one prong extending from the base portion, the at least one prong being configured to direct the gases flow to an orifice of the patient, and
one or more sensors configured to measure a parameter,
wherein the sensor is configured to contact the neck of the patient when the nasal cannula interface is in use.

### BRIEF DESCRIPTION OF THE DRAWINGS

Throughout the drawings, reference numbers can be reused to indicate general correspondence between reference elements. The drawings are provided to illustrate example embodiments described herein and are not intended to limit the scope of the disclosure.
**Figure 1** shows in diagrammatic form a respiratory support apparatus.
**Figure 2** shows a schematic diagram of a closed loop control system for use with the respiratory support apparatus of Figure 1.
**Figure 3** shows a nasal cannula in use with a patient in accordance with an aspect of this disclosure.
**Figure 4** shows a partial front view of the nasal cannula in accordance with an aspect of this disclosure.
**Figure 5** shows an exploded view of the nasal cannula of Figure 4.
**Figure 6** shows a front view of a body of the nasal cannula in accordance with an aspect of this disclosure.
**Figure 7** shows a rear view of the body of the nasal cannula of Figure 6 including a patient sensor.
**Figure 8** shows a rear view of the body of the nasal cannula of Figure 6 including an alternative placement of the patient sensor.
**Figure 9** shows a rear view of the body of the nasal cannula of Figure 6 including a further alternative placement of the patient sensor.
**Figure 10** shows a view directly from the rear of the body of the nasal cannula of Figure 6 including a further alternative placement of the patient sensor.
**Figure 11** shows a front perspective view of an alternative configuration of the body of the nasal cannula including facial pads in accordance with an aspect of this disclosure.
**Figure 12** shows a view directly from the rear of the body of the nasal cannula of Figure 11.
**Figure 13** shows a front perspective view of an alternative configuration of the body of the nasal cannula including two-part facial pads in accordance with an aspect of this disclosure.
**Figure 14** shows a side view of the nasal cannula of Figure 13.
**Figure 15** shows an exploded view of the nasal cannula of Figure 13.
**Figure 16** shows the nasal cannula in use with the patient including an alternative configuration of the patient sensor in accordance with an aspect of this disclosure.
**Figure 17** shows an enlarged view of section B of Figure 16.
**Figure 18** shows the nasal cannula coupled to the patient including an alternative configuration of the patient sensor in accordance with an aspect of this disclosure.
**Figure 19** shows an enlarged view of section B of Figure 15.
**Figure 20** shows the nasal cannula in use with the patient including an alternative configuration of the patient sensor in accordance with an aspect of this disclosure.
**Figure 21** shows an enlarged view of section B of Figure 19.
**Figure 22** shows a possible range of movement of the patient sensor of Figures 20 and 21.
**Figure 23** shows a further possible range of movement of the patient sensor of Figures 20 and 21.
**Figure 24** shows a further possible range of movement of the patient sensor of Figures 20 and 21.
**Figure 25** shows a further possible range of movement of the patient sensor of Figures 20 and 21.
**Figures 26 to 28** show the nasal cannula in use with the patient including an alternative placement of the wire coil in accordance with an aspect of this disclosure.
**Figure 29** shows an enlarged view of section D of Figure 27.
**Figures 30 to 32** show the nasal cannula in use with the patient including a wire coil in accordance with an aspect of this disclosure.
**Figure 33** shows the nasal cannula in use with the patient including the patient sensor on a forehead strap in accordance with an aspect of this disclosure.
**Figure 34** shows a mask assembly in use with the patient including the patient sensor on a forehead strap in accordance with an aspect of this disclosure.
**Figure 35** shows a tracheostomy interface in use with the patient including the patient sensor on a neck strap in accordance with an aspect of this disclosure.
**Figure 36** shows the tracheostomy interface in use with the patient including the patient sensor on a neck band in accordance with an aspect of this disclosure.
**Figures 37A** **and 36B** respectively show perspective and perspective exploded views of another nasal cannula in accordance with this disclosure, whilst **Figure 37B** shows a perspective view of headgear of the nasal cannula.
**Figures 38A and 38B** show enlarged perspective views of the connection between the nasal cannula and the headgear, showing preferred forms of a sleeve of the headgear.
**Figures 39A to 39B** show enlarged perspective views of a retention clip of the nasal cannula in accordance with this disclosure.
**Figures 40A to 40C** show enlarged perspective views of a headgear connector connecting to a nasal cannula in accordance with this disclosure.
**Figures 41A and 41B** respectively show cross-sectional views corresponding to Figures 40A and 40B.
**Figure 42** is a perspective view of another embodiment of a nasal cannula in accordance with this disclosure.
**Figure 43** is a perspective view of a manifold part of the nasal cannula of Figure 42.

### DETAILED DESCRIPTION

Certain embodiments and examples of a respiratory support system, and patient interfaces for such a system, are described herein. Those of skill in the art will appreciate that the scope of the claims extends beyond the specifically disclosed embodiments. Thus, it is intended that the scope of the disclosure herein disclosed should not be limited by any particular embodiments described herein.

Patients suffering from various health conditions and diseases can benefit from respiratory support. For example, patients suffering from conditions such as chronic obstructive pulmonary disease (COPD), pneumonia, asthma, bronchopulmonary dysplasia, heart failure, cystic fibrosis, sleep apnea, lung disease, trauma to the respiratory system, acute respiratory distress, receiving pre- and post- operative oxygen delivery, and other conditions or diseases can benefit from respiratory support. As a part of providing a patient with respiratory support, one or more physiological parameters of the patient may be measured by a patient sensor for the purpose of monitoring the patient's health. The patient sensor may be a pulse oximeter, which provides information relating to heart rate and blood oxygen saturation (SpO₂).

When providing a patient with respiratory support, in particular supplemental oxygen therapy, a common method of monitoring the patient's health is to ensure that their SpO₂ does not drop too low (e.g., typically below about 90%).

However, supplying the patient with too much oxygen can over oxygenate their blood, and is also considered dangerous. Generally, the patient's SpO₂ is kept in a range from about 80% to about 99%, and preferably about 92% to about 96%, although these ranges may differ due to patient conditions, and/or from patient to patient.

Due to various patient factors such as respiratory rate, lung tidal volume, heart rate, activity levels, height, weight, age, gender, and other factors, there is no one prescribed level of supplemental oxygen that can consistently achieve an SpO₂ response in the targeted range for each patient. Individual patients regularly need their fraction of oxygen delivered to the patient (FdO₂) monitored and adjusted to ensure they are receiving the correct FdO₂ to achieve the targeted SpO₂. Achieving a correct and consistent SpO₂ is an important factor in treating patients with various health conditions or diseases. Additionally, patients suffering from these health problems may find benefit from a system that automatically controls oxygen saturation. The present disclosure is applicable to a wide range of patients that require fast and accurate oxygen saturation control.

The fraction of oxygen delivered to a patient (FdO₂) may be controlled manually. For example, a user can manually adjust an oxygen supply valve to change the flow rate or fraction of oxygen being delivered to the patient. The user can determine SpO₂ levels of the patient using a patient monitor, such as a pulse oximeter. The SpO₂ measurements can be displayed on respiratory support apparatus 10 or on the pulse oximeter itself. The user can continue to manually adjust the amount of oxygen being delivered to the patient until the SpO₂ level of the patient reaches a determined level.

When a patient sensor is used as a part of the respiratory support system, the user is required to mount the patient sensor to the patient. This adds another task required of the user to an already potentially large set of tasks required for setting up the respiratory support system. Additionally, the separate patient sensor can lead to problems such as incorrect mounting of the sensor leading to incorrect measurement and/or the sensor falling off during use.

As such, a patient interface which incorporates the patient sensor allows the use of the patient sensor without increasing the workload of the user. This may have benefits in a hospital setting, in which a single clinician might have a large group of patients for whom they need to address. Additionally, this may have benefits in a home setting, as it simplifies the setup process for a patient who may need to perform these tasks themselves. Furthermore, integrating the patient sensor into the patient interface helps ensure correct orientation of the patient sensor and prevents the patient sensor from falling off during use.

This disclosure references conduit heaters, which is a broad term and is to be given its ordinary and customary meaning to a person of ordinary skill in the art (that is, it is not to be limited to a special or customized meaning) and includes, without limitation, one or more heater strips, one or more heater wires, and/or one or more conductive elements that produce heat when electrical power is provided. Examples of such conduit heaters include wires made of a conductive metal *(e.g.,* copper), conductive polymers, conductive inks printed on a surface of a conduit, conductive materials used to create a track on a conduit, and the like.

Furthermore, the disclosure references conduits, limbs, and medical conduits in the context of gas delivery. Conduit, for example, is a broad term and is to be given its ordinary and customary meaning to a person of ordinary skill in the art and includes, without limitation, passageways having a variety of cross-sections such as cylindrical and non-cylindrical passageways.

The disclosed systems, apparatuses and medical conduits can also be used in breathing circuits configured to provide a continuous, variable, or bi-level positive airway pressure (PAP) therapy or other form of respiratory support such as high flow or low flow oxygen therapy. The breathing circuit may for example comprising an inspiratory circuit which at a minimum includes the inspiratory gases pathway (including all the components) from the gases supply to the patient interface.

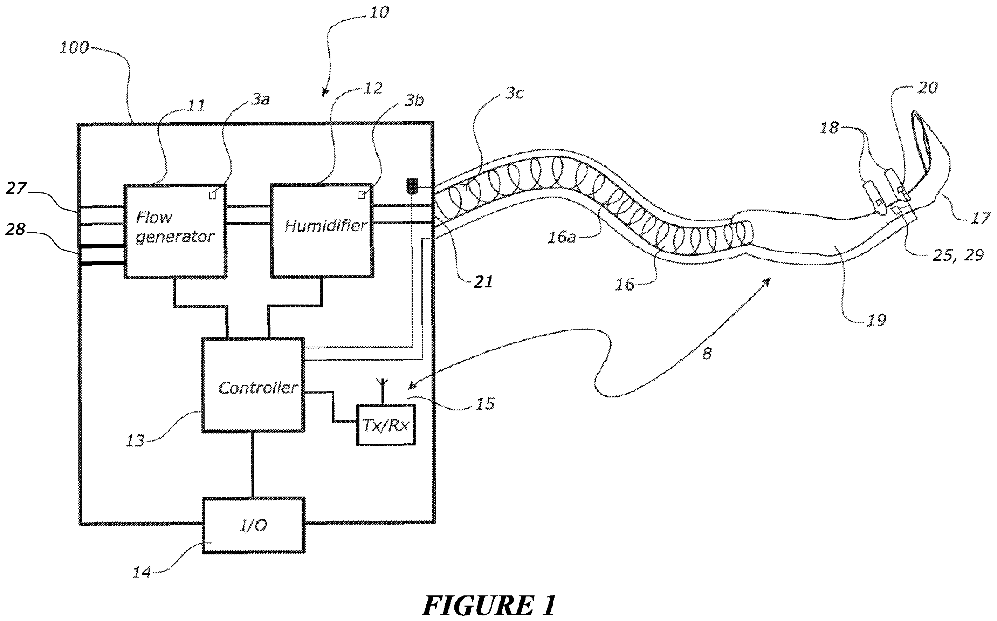

A respiratory support system may comprise a respiratory support apparatus 10 and the patient interface with an integrated sensor as shown in Figure 1. The respiratory support apparatus 10 may be configured to provide high flow therapy for example. The respiratory support apparatus 10 may comprise a main housing 100 that contains a flow generator 11, such as in the form of a motor/impeller arrangement (for example, a blower), an optional humidifier 12, a controller 13, and a user interface 14 (comprising, for example, a display and input device(s) such as button(s), a touch screen, or the like). The humidifier may comprise a heater bay comprising a heating element such as a heater plate and configured to receive a humidification chamber. In use the humidification chamber receives heat from the heating element to raise the temperature of a body of water contained within the humidification chamber. The gases flow is passed over the body of water to add heat and humidity to the gases flow.

The controller 13 can be configured or programmed to control the operation of the apparatus. For example, the controller can control components of the apparatus, including but not limited to: operating the flow generator 11 to create a flow of gas (gases flow) for delivery to a patient, operating the humidifier 12 (if present) to humidify and/or heat the generated gases flow, control a flow of oxygen into the flow generator, receiving user input from the user interface 14 for reconfiguration and/or user-defined operation of the apparatus 10, and outputting information (for example on the display) to the user. The user can be a patient, healthcare professional, or anyone else interested in using the apparatus. As used herein, a "gases flow" can refer to any flow of gases that may be used in the breathing assistance or respiratory support apparatus 10, such as a flow of ambient air, a flow comprising substantially 100% oxygen, a flow comprising some combination of ambient air and oxygen, and/or the like.

An inspiratory conduit 16 is coupled at one end to a gases flow outlet 21 in the housing 100 of the respiratory support apparatus 10. In an alternative configuration, the inspiratory conduit 16 is coupled at one end to a gases flow outlet of the humidifier 12. The inspiratory conduit 16 is coupled at another end to a patient interface 17 such as a non-sealed nasal cannula with a body 19 comprising one or more nasal prongs 18. Additionally, or alternatively, the inspiratory conduit 16 can be coupled to a face mask, a nasal mask, a nasal pillows mask, an endotracheal tube, a tracheostomy interface, and/or the like. The gases flow that is generated by the respiratory support apparatus 10 may be humidified and delivered to the patient via the inspiratory conduit 16 through the cannula 17. The inspiratory conduit 16 can have a conduit heater such as one or more heater wires 16a to heat gases flow passing through to the patient. The conduit heater can be under the control of the controller 13. The respiratory support apparatus 10, inspiratory conduit 16, and patient interface 17 together can form a respiratory support system.

The controller 13 can control the flow generator 11 to generate a gases flow of the desired flow rate. The controller 13 can also control a supplemental oxygen inlet to allow for delivery of supplemental oxygen, the humidifier 12 (if present) can humidify the gases flow and/or heat the gases flow to an appropriate level, and/or the like. The gases flow is directed out through the inspiratory conduit 16 and cannula 17 to the patient. The controller 13 can also control a heating element in the humidifier 12 and/or the heating element 16a in the inspiratory conduit 16 to heat the gas to a desired temperature for a desired level of therapy and/or level of comfort for the patient. The controller 13 can be programmed with or can determine a suitable target temperature of the gases flow.

The oxygen inlet port 28 can include a valve through which a pressurized gas may enter the respiratory support apparatus 10. The valve can control a flow of oxygen into the respiratory support apparatus 10. The valve can be any type of valve, including a proportional valve or a binary valve. The source of oxygen can be an oxygen tank or a hospital oxygen supply. Medical grade oxygen is typically between 95% and 100% purity. Oxygen sources of lower purity can also be used. Examples of valve modules and filters are disclosed in U.S. Provisional Application No. 62/409,543, titled "Valve Modules and Filter", filed on October 18, 2016, and U.S. Provisional Application No. 62/488,841, titled "Valve Modules and Filter", filed on April 23, 2017.

The respiratory support apparatus 10 can measure and control the oxygen content of the gas being delivered to the patient, and therefore the oxygen content of the gas inspired by the patient. During high flow therapy, the high flow rate of gas delivered meets or exceeds the peak inspiratory demand of the patient. This means that the volume of gas delivered by the respiratory support apparatus 10 to the patient during inspiration meets, or is in excess of, the volume of gas inspired by the patient during inspiration. High flow therapy therefore helps to prevent entrainment of ambient air when the patient breathes in, as well as flushing the patient's airways of expired gas. So long as the flow rate of delivered gas meets or exceeds peak inspiratory demand of the patient, entrainment of ambient air is prevented, and the gas delivered by the respiratory support apparatus 10 is substantially the same as the gas the patient breathes in. As such, the oxygen concentration measured in the respiratory support apparatus 10, fraction of delivered oxygen, (FdO₂) is substantially the same as the oxygen concentration the user is breathing, fraction of inspired oxygen (FiO₂), and as such the terms may can be seen as equivalent.

Operation sensors 3a, 3b, 3c, such as flow, temperature, humidity, and/or pressure sensors can be placed in various locations in the respiratory support apparatus 10. Additional sensors (for example, sensors 20, 25) may be placed in various locations on the inspiratory conduit 16 and/or cannula 17 (for example, there may be a temperature sensor at or near the end of the inspiratory conduit 16). The sensors 20, 25 may also be a CO2 sensor or a pressure sensor or a flow sensor or oxygen sensor. Output from the sensors can be received by the controller 13, to assist the controller in operating the respiratory support apparatus 10 in a manner that provides suitable therapy. In some configurations, providing suitable therapy includes meeting a patient's peak inspiratory demand. The apparatus 10 may have a transmitter and/or receiver 15 to enable the controller 13 to receive signals 8 from the operation and any additional sensors and/or to control the various components of the respiratory support apparatus 10, including but not limited to the flow generator 11, humidifier 12, and heater wire 16a, or accessories or peripherals associated with the respiratory support apparatus 10. Additionally, or alternatively, the transmitter and/or receiver 15 may deliver data to a remote server or enable remote control of the apparatus 10.

The respiratory support apparatus 10 may receive measurements from one or more gases composition sensors. The gases composition sensors may be located within the respiratory support apparatus 10, the inspiratory conduit 16, the patient interface, or at any other suitable location. The gases composition sensors may be located at or downstream of a location where the ambient air and any supplemental gases flow, such as oxygen, have finished mixing. The gases composition sensors may be configured to measure oxygen concentration. The gases composition sensors may be an ultrasonic transducer system, also referred to herein as an ultrasonic sensor system.

The respiratory support apparatus 10 may be configured to connect to a patient sensor 29 as described below, such as a pulse oximeter or a patient monitoring system, to measure one or more physiological parameters of the patient, such as a patient's blood oxygen saturation (SpO₂) (i.e. peripheral arterial oxyhaemoglobin), heart rate, respiratory rate, perfusion index, and provide a measure of signal quality. The patient sensor 29 may be part of the additional sensors 20, 25 or may be a separate additional sensor that could be located on or in the patient interface or delivery conduit. The sensor can communicate with the controller 13 through a wired connection or by communication through a wireless transmitter on the sensor. Sensors are available that are designed for different age groups and to be connected to different locations on the patient, which can be used with the respiratory support apparatus.

The pulse oximeter connects to a processor in the respiratory support apparatus 10 and constantly provides signals indicative of the patient's blood oxygen saturation. The patient sensor 29 may be a hot swappable device. The term "hot swappable device" as used herein refers to a device that can be attached or interchanged during operation of the respiratory support apparatus 10. For example, the patient sensor 29 may connect to the respiratory support apparatus 10 using a USB interface with a lead or wire or using wireless communication protocols (such as, for example, near field communication, WiFi or Bluetooth^{®}). The output of the pulse oximeter may be displayed on the graphical user interface 14. The measurements from the pulse oximeter 29 may also be transmitted to a remote patient management system (e.g. a remote server system) via a suitable wireless protocol e.g. via GSM etc.

When the patient sensor 29 is disconnected (either from the patient or from the respiratory support apparatus) during operation, the respiratory support apparatus 10 may continue to operate in its previous state of operation for a predefined time period. After the predefined time period, the respiratory support apparatus 10 may trigger an alarm, transition from automatic mode to manual mode, and/or exit control mode (e.g., automatic mode or manual mode) entirely.

The respiratory support apparatus 10 may be configured to recognise whether patient sensor 29 is a standalone patient sensor or a patient sensor that is located on or comprised by a patient interface 17. The respiratory support apparatus 10 may recognise the sensor type by receiving identification information upon initial connection of the patient sensor 29. The respiratory support apparatus 10 may recognise the sensor type through the way in which the signal from the patient sensor 29 is received. For example, an integrated patient sensor 29 may be configured to communicate with the respiratory support apparatus 10 via an electrical connection located at the gases outlet of the respiratory support apparatus 10 (as will be described herein), whilst a standalone patient sensor may be configured to connect to the respiratory support apparatus via a separate connection port.

The respiratory support apparatus 10 may be configured to use the output of a patient sensor 29 that is located on or comprised by a patient interface 17 to determine whether the patient is wearing the patient interface 17. In this context, "wearing" refers to the patient interface 17 being mounted in a position on the patient's face such that the patient interface 17 can deliver the gases flow to the patient and the patient sensor 29 can measure one or more patient parameters. When the patient sensor 29 cannot reliably measure the one or more patient parameters it may produce a signal indicating such. Additionally, or alternatively, the patient sensor 29 may communicate a separate parameter such as signal quality. The respiratory support apparatus 10 may check this parameter against a threshold in determining whether the patient sensor 29 is able to reliably measure the one or more patient parameters. The respiratory support apparatus 10 may use a determination that the patient sensor 29 cannot reliably measure the one or more patient parameters in further determining that the patient is not wearing the patient interface 17.

The respiratory support apparatus 10 may use the determination of whether the patient is wearing the patient interface 17 in activating or deactivating certain control algorithms, such as the closed loop SpO₂ controller, which will be described in detail later in the specification. The respiratory support apparatus 10 may use the indication in increasing or decreasing the flow rate. For example, the respiratory support apparatus 10 may reduce the flow rate when the patient is not wearing the patient interface 17 in order to reduce noise and power consumption. The respiratory support apparatus 10 may use the indication for generating an alarm, such as alarming if the patient has removed the patient interface 17. This alarm may occur instantaneously or within a set period of time after the output of the patient sensor 29 is lost.

In a further configuration, the respiratory support apparatus 10 is configured to switch to a standby mode when the output of the patient sensor 29 indicates that the patient is not wearing the patient interface 17. In the standby mode, the respiratory support apparatus 10 may be configured to control the blower to operate at a reduced motor speed. The reduced motor speed may be a minimum operating speed for the blower. The reduced motor speed may be about 1000RPM - 2000RPM. In the standby mode, the respiratory support apparatus 10 may be configured to control a blower to deliver a reduced flow rate. The reduced motor speed may be between about 1 LPM and 2 LPM.

The respiratory support apparatus 10 may comprise a high respiratory support apparatus. High flow therapy as discussed herein is intended to be given its typical ordinary meaning as understood by a person of skill in the art, which generally refers to a respiratory assistance system delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute (LPM) to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. The flow rates used to achieve "high flow" may be any of the flow rates listed below. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high respiratory support apparatus with an adult patient, a neonatal, infant, or child patient, may deliver gases to the patient at a flow rate of between about 1 LPM and about 100 LPM, or at a flow rate in any of the sub-ranges outlined above. The respiratory support apparatus 10 can deliver any concentration of oxygen (e.g., FdO₂), up to 100%, at any flow rate between about 1 LPM and about 100 LPM. In some configurations, any of the flow rates can be in combination with oxygen concentrations (FdO₂s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO₂) of about 20%-30%, 21%-30%, 21%-40%, 30%-40%, 40%-50%, 50%-60%, 60%-70%, 70%-80%, 80%-90%, and 90%-100%. In some configurations, the respiratory support apparatus 10 may include safety thresholds when operating in manual mode that prevent a user from delivering to much oxygen to the patient.

High flow therapy may be administered to the nares of a user and/or orally, or via a tracheostomy interface. High flow therapy may deliver gases to a user at a flow rate at or exceeding the intended user's peak inspiratory flow requirements. The high flow therapy may generate a flushing effect in the nasopharynx such that the anatomical dead space of the upper airways is flushed by the high incoming gases flow. This can create a reservoir of fresh gas available for each and every breath, while minimizing rebreathing of nitrogen and carbon dioxide. Meeting inspiratory demand and flushing the airways is additionally important when trying to control the patient's FdO₂. High flow therapy can be delivered with a non-sealing patient interface such as, for example, a nasal cannula. The nasal cannula may be configured to deliver breathing gases to the nares of a user at a flow rate exceeding the intended user's peak inspiratory flow requirements.

The term "non-sealing patient interface" as used herein can refer to an interface providing a pneumatic link between an airway of a patient and a gases flow source (such as from flow generator 11) that does not completely occlude the airway of the patient. Non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or more of a nare or mouth of the patient. For a nasal cannula the airway is through the nares.

The respiratory support apparatus 10 can include an ambient air inlet port 27 to entrain ambient room air. The respiratory support apparatus 10 may also include an oxygen inlet port 28 leading to a valve through which a pressurized gas may enter the respiratory support apparatus 10. The valve can control a flow of oxygen into the respiratory support apparatus 10. The valve can be any type of valve, including a proportional valve or a binary valve.

In a further configuration, the respiratory support apparatus 10 includes two or more oxygen inlet ports. A first oxygen inlet port, also referred to as a high pressure oxygen inlet, receives oxygen from an oxygen source at a set pressure. The flow rate of the oxygen from the first oxygen inlet port is then regulated by a valve as described above. A second oxygen inlet port, also referred to as a low pressure oxygen inlet, receives oxygen from an oxygen source at a set flow rate. The flow rate of oxygen through the second oxygen inlet port can then be adjusted by adjusting an external flow regulator on the oxygen source.

The blower can operate at a motor speed of greater than about 1,000 RPM and less than about 30,000 RPM, greater than about 2,000 RPM and less than about 21,000 RPM, greater than about 4,000 RPM and less than about 19,000 RPM or between any of the foregoing values. Operation of the blower can mix the gases flow entering the blower through the inlet ports. Using the blower as the mixer can decrease the pressure drop that otherwise occurs in a system with a separate mixer, such as a static mixer comprising baffles, because mixing requires energy. Having a static mixer can also increase the volume of the gas flow path between the valve and the gases composition sensor, which can further increase the delay between when the valve current is changed and when a corresponding change in oxygen concentration is measured.

Based on user inputs and the therapy supplied by the respiratory support apparatus 10, the controller 13 can determine a target output parameter for the blower. The controller can receive measurements of the target output parameter, and based on the difference between determined flow rate and the measured flow rate, the controller can adjust the speed of the blower.

With reference again to Figure 1, the controller 13 can be programmed with or configured to execute a closed loop control system for controlling the operation of the respiratory support apparatus. The closed loop control system can be configured to ensure the patient's SpO₂ reaches a target level and consistently remains at or near this level.

The controller 13 can receive input(s) from a user that can be used by the controller 13 to execute the closed loop control system. The target SpO₂ value can be a single value or a range of values. The value(s) may be pre-set, chosen by a clinician, or determined based on the type of patient, where type of patient may refer to current affliction, and/or information about the patient such as age, weight, height, gender, and other patient characteristics. The target SpO₂ value may be input by a clinician or user via a user interface on the apparatus and received by the controller 13. Similarly, the target SpO₂ may be two values, each selected in any way described above. The two values represent a range of acceptable values for the patient's SpO₂. The controller can target a value within said range. The targeted value may be the middle value of the range, or any other value within the range, which may be pre-set or selected by a user. Alternatively, the range may be automatically set based on the targeted value of SpO₂. The controller can be configured to have one or more set responses when the patient's SpO₂ value moves outside of the range. The responses may include alarming, changing to manual control of FdO₂, changing the FdO₂ to a specific value, and/or other responses. The controller can have one or more ranges, where one or more different responses occur as it moves outside of each range.

Generally, SpO₂ is controlled between about 80% and about 100%, or about 80% and about 90%, or about 88% and about 92%, or about 90% and about 99%, or about 92% and about 96%. The SpO₂ may be controlled between any two suitable values from any two of the aforementioned ranges. The target SpO₂ may be between about 80% and about 100%, or between about 80% and about 90%, or between about 88% and about 92%, or between about 90% and about 99%, or between about 92% and about 96%, or about 94%, or 94% or about 90%, or 90%, or about 85%, or 85%. The SpO₂ target may be any value between any two suitable values from any two of the aforementioned ranges. The SpO₂ target can correspond to the middle of the SpO₂ for a defined range.

The FdO₂ can be configured to be controlled within a range. As discussed previously, the oxygen concentration measured in the apparatus (FdO₂) is substantially the same as the oxygen concentration the patient is breathing (FiO₂) so long as the flow rate meets or exceeds the peak inspiratory demand of the patient, and as such the terms may can be seen as equivalent. Each of the limits of the range may be pre-set, selected by a user, or determined based on the type of patient, where the type of patient may refer to current affliction, and/or information about the patient such as age, weight, height, gender, and/or other patient characteristics. Alternatively, a single value for FdO₂ may be selected, and the range may be determined at least partially based on this value. For example, the range may be a set amount above and below the selected FdO₂. The selected FdO₂ may be used as the starting point for the controller. The system may have one or more responses if the controller tries to move the FdO₂ outside of the range. These responses may include alarming, preventing the FdO2 moving outside of the range, switching to manual control of FdO₂, and/or switching to a specific FdO₂. The respiratory support apparatus 10 may have one or more ranges where one or more different responses occur as it reaches the limit of each range.

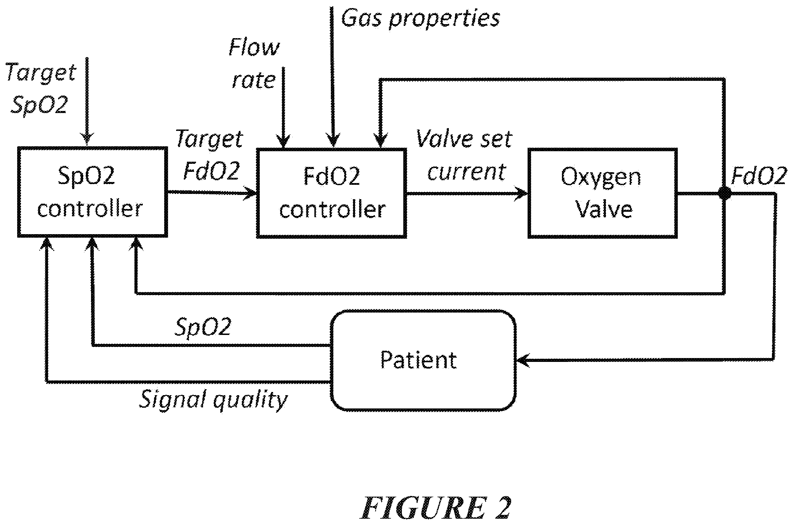

With reference to Figure 2 a schematic diagram of the closed loop control system 1000 is illustrated. The closed loop control system may utilize two control loops. The first control loop may be implemented by the SpO₂ controller. The SpO₂ controller can determine a target FdO₂ based in part on the target SpO₂ and/or the measured SpO₂. As discussed above, the target SpO₂ value can be a single value or a range of acceptable values. The value(s) may be pre-set, chosen by a clinician, or determined automatically based on client characteristics. Generally, target SpO₂ values are received or determined before or at the beginning of a therapy session, though target SpO₂ values may be received at any time during the therapy session. During a therapy session, the SpO₂ controller can also receive as inputs: measured FdO₂ reading(s) from a gases composition sensor, and measured SpO₂ reading(s) and a signal quality reading(s) from the patient sensor 29. In some configurations, the SpO₂ controller can receive target FdO₂ as an input, in such a case, the output of the SpO₂ controller may be provided directly back to the SpO₂ controller as the input. Based at least in part on the inputs, the SpO₂ controller can output a target FdO₂ to the second control loop.

During the therapy session, the SpO₂ and FdO₂ controllers can continue to automatically control the operation of the respiratory support apparatus until the therapy session ends or an event triggers a change from the automatic mode to manual mode.

For example, a respiratory support system using blood oxygen saturation measurements from a pulse oximeter to automatically adjust the fraction of oxygen of the gases flow being delivered to a patient via a patient interface is described in our earlier PCT application WO2019/070136 (herein WO' 136) filed on 5 October 2018.

The respiratory support system described in WO' 136 uses a separate pulse oximeter and patient interface. As such, a clinician is required to attach both the pulse oximeter and the patient interface to the patient individually, with both of these components also being connected separately to a respiratory support apparatus.

With reference again to Figure 1, the controller 13 can be programmed with or configured to execute an FdO₂ control system for controlling the operation of the respiratory support apparatus.

The FdO₂ control system can be configured to ensure that the instantaneous FdO₂ is maintained at a target level at all points during a therapy session. The controller can measure the FdO₂, compare it with the target FdO₂, and then adjust the oxygen inlet valve accordingly. However, when the FdO₂ sensors are located at a non-insignificant distance away from the valve, there is a time delay between when a change is made to the valve and when a corresponding change in the FdO₂ is measured. The controller may adjust the valve after the time delay. However, if the flow rate is fluctuating, then the controller may be able to achieve the target FdO₂ on average, but not at a continuous and substantially instantaneous basis. As shown in Figure 2, in order to maintain the FdO₂ at the target level at a continuous and substantially instantaneous basis, without moving the FdO₂ sensors closer to the valve, the FdO₂ controller can factor in the measurement of a total flow rate into the control of the valve.

A patient interface 17 is connected to one end of the inspiratory conduit 16 and is used to provide a breathable gases flow to the patient. During setup of the respiratory support apparatus 10, a clinician or the patient is required to attach the patient interface 17 to the patient. Additionally, if a standalone patient sensor 29 is also to be used, then the clinician or the patient are also required to attach this to the patient. Both of the patient interface 17 and the patient sensor 29 then also need to be attached to the respiratory support apparatus 10 itself. Forming these various connections can be undesirable.

The patient interface 17 has one or more patient sensors 29. The one or more integrated patient sensors 29 may be configured to measure the blood oxygen saturation of the patient. The one or more integrated patient sensors 29 are positioned on the patient interface 17 to facilitate the measuring of the patient's blood oxygen saturation.

The patient interface 17 may be used with the respiratory support apparatus 10 described above. Alternatively, the patient interface 17 may be used with any other respiratory support apparatus that may utilize a patient interface 17 with patient sensors 29, such as a ventilator, a CPAP apparatus, a standalone humidifier, and/or an oxygen blender.

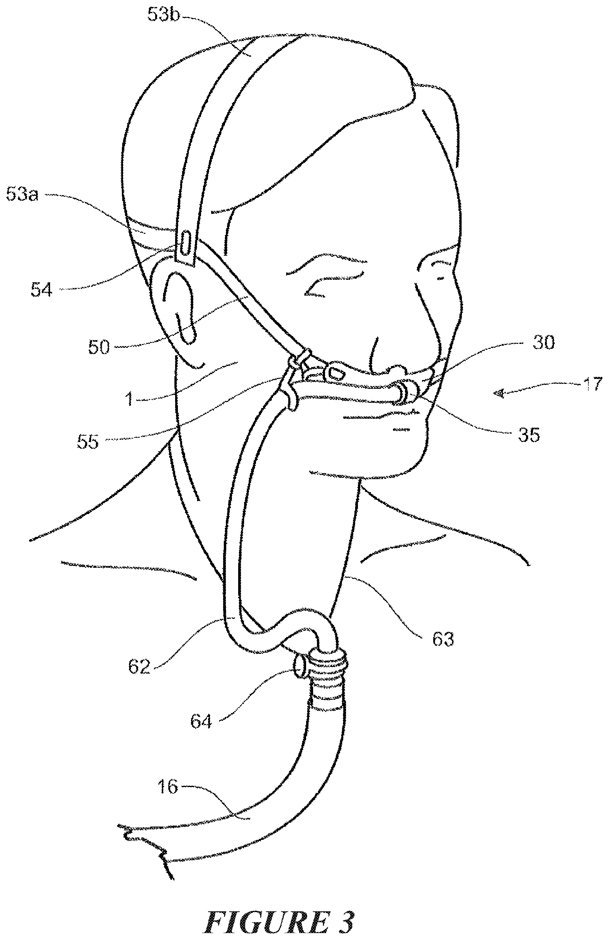

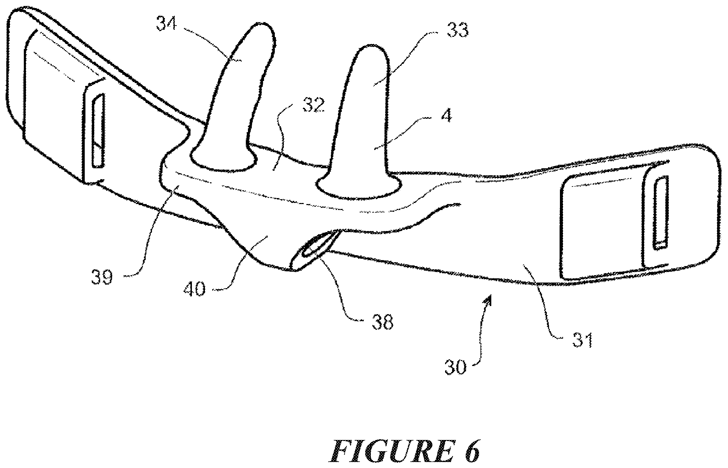

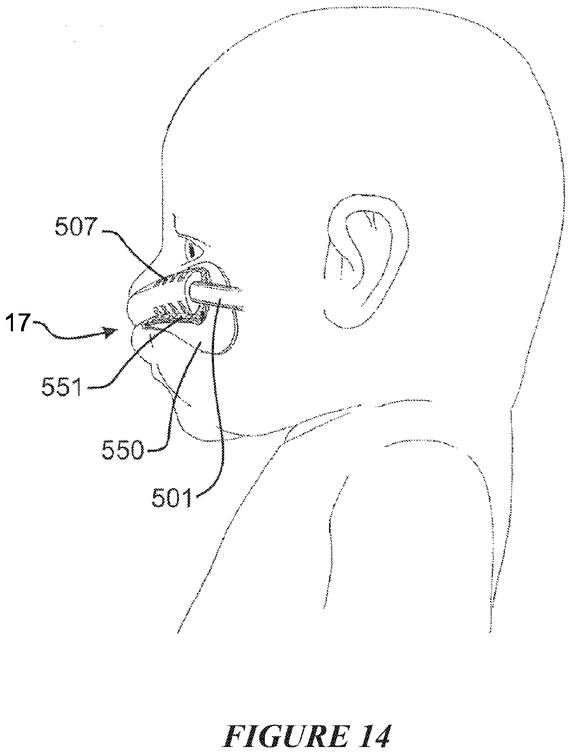

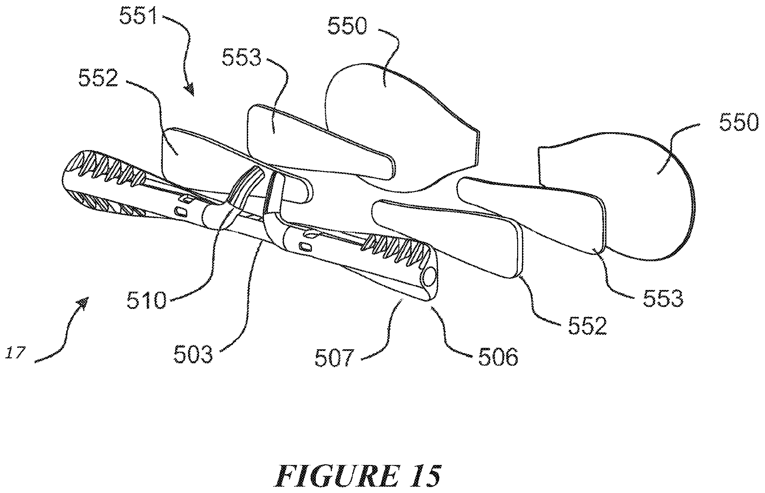

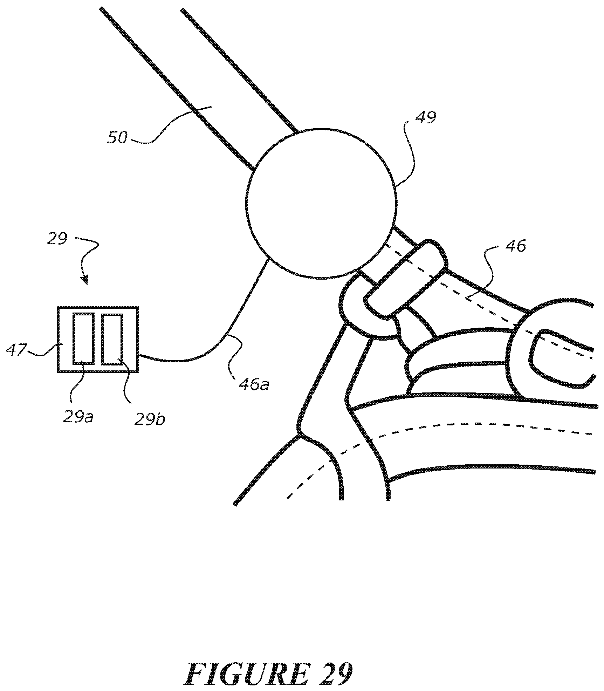

The patient interface 17 may comprise a nasal cannula interface, as shown in Figures 3 to 33. In this configuration, the nasal cannula interface broadly comprises a head securement assembly and a nasal cannula 30, and also includes a gases inlet conduit 62. The head securement assembly enables a user to place and maintain the nasal cannula 30 in the correct operational position. The gases inlet conduit 62 forms a fluid or gases connection between the outlet end of the inspiratory conduit 16 and the nasal cannula 30 to allow fluids or gases to flow between the inspiratory conduit and nasal cannula. The gases inlet conduit 62 and detail of the main portion of the nasal cannula 30 will be described in detail below.

The head securement assembly of the nasal cannula 30 may comprise one or more straps. The one or more straps may include two front straps 50, a rear strap 53a, and a top strap 53b, as shown in Figure 3. In some configurations, the proximal end of the front straps 50 are removably connected to the nasal cannula 30. In other configurations, the proximal ends of the front straps 50 are non-removably connected to the nasal cannula 30. The rear strap 53a and the top strap 53b extend between the distal ends of the front straps 50. In use, the rear strap 53a wraps around the back of the patient's head. In use, the top strap 53b wraps around the top of the patient's head. In some configurations the head securement assembly is adjustable to allow patients of different head shapes and sizes to use the nasal cannula 30. For example, an adjuster such as an adjustment buckle 54 may be included which allows a patient to loosen or tighten the top strap 53b.

In some configurations, one or more of the straps are substantially elastic (i.e. made from an elastic material e.g. lycra, that can stretch to accommodate a patient's head). In some configurations, one or more of the straps are substantially rigid. In some configurations, one or more of the straps are made of a substantially rigid material. In some configurations, one or more of the straps are substantially inextensible. In some configurations, one or more of the straps are made of a substantially inextensible material. In some configurations, one or more of the straps are self-supporting. In some configurations, one or more of the straps maintain their shape when not in use.

Alternatively, the patient interface 17 is secured to the patient's head and face by front straps 50 and a single rear strap 53a attached to the front straps 50. The rear strap is attached to the front straps 50 via a buckle 54. Alternatively, the rear strap 53a is integral with the front straps 50. The buckle 54 allows a patient to loosen or tighten the front straps 50 based on individual preference. Alternatively, the integral front 50 and rear straps 53a are elastic and can be stretched over a patient's head. The elasticity of the straps exerts a force upon the head to hold the nasal cannula 30 in the optimal position when in use. Elastic straps 50, 53a can be used with the adjustment buckle 54 or the elastic straps 50, 53a may be used on their own without the buckle 54.

The head securement assembly may also include a loop 55 which holds and supports the gases inlet conduit 62 at or close to the inlet end as shown in Figure 3. The loop 55 comprises a first end connected to one of the front straps 50. The first end may be slidably connected to the front strap 50. The loop 55 comprises a second end connected to the gases inlet conduit 62. The second end may be removably connected to the gases inlet conduit 62. Alternatively, the interface may comprise a tube clip that is connected to the tube and can be removably coupled to the cannula. The tube clip supports the weight of the inlet conduit 62 and reduces the moment cause by the conduit 62, thereby improving stability of the patient interface 17. The clip helps to reduce dislodgement of the patient interface 17. The clip may be formed of a rigid material.

A lanyard 63 may also be provided with the patient interface 17. Figure 3 shows an example of a lanyard 63. In the configuration shown, the lanyard 63 is connected to the gases inlet conduit 62. Alternatively, the lanyard 63 is connected at a location at or close to the connection between the inspiratory conduit 16 and the gases inlet conduit 62. In use, the lanyard 63 supports the weight of the inspiratory conduit 16 and the gases inlet conduit 62. A toggle 64 is provided with the lanyard 63 to allow adjustment of the lanyard's length. The toggle 64 makes the lanyard 63 suitable for any sized patient to use the patient interface 17. The lanyard 63 supports at least a portion of the weight of the inspiratory conduit 16 in use, such that the weight does not act on the user or the nasal cannula 30. The use of the lanyard 63 reduces the portion of the combined weight of the inspiratory conduit 16 and the gases inlet conduit 62 that pulls on the nasal cannula 30, helping to prevent the nasal prongs 33, 34 from interfering with the sensitive lining of the nasal passages, or becoming displaced or misaligned in use. In the configuration shown the lanyard 63 is loose fitting around the neck so as to reduce the chance of strangulation of the user. The lanyard 63 also provides a convenient way of supporting the inspiratory conduit 16 and the gases inlet conduit 62. This allows the patient to turn in bed without tugging or pulling on the inspiratory conduit 16 and helps avoid having the gases inlet conduit 62 from overheating under the blankets. In one configuration the lanyard 63 has a clip that allows the lanyard to be opened and closed by a user in order place and secure the lanyard 63 around a user's neck. The clips comprises a male and female connector that snap fit together. The clip is disconnected by pulling one end of the lanyard 63. The clip is easily disconnectable, and uncouples when the user pulls on one side of the lanyard. This allows the lanyard 63 to be removed quickly, for example in an emergency situation, such as if the patient needs to be intubated.

The gases inlet conduit 62 will now be described in detail. The gases inlet conduit 62 is a short length of conduit or tubing relative to the inspiratory conduit 16 which runs between the outlet of the inspiratory conduit 16 and the nasal cannula 30. In use, the gases inlet conduit 62 forms a lumen that defines a gases pathway between the inspiratory conduit 16 and the patient interface 17, such that the gases flow exits the inspiratory conduit 16 and enter the gases inlet conduit 62, travelling along the gases inlet conduit 62 to the patient interface 17 to be delivered to the patient. One reason that secondary conduits such as the gases inlet conduit 62 can be used is as follows: the inspiratory conduit 16 is relatively heavy and cumbersome as it is used to transport the gases flow over a reasonably long distance (from the humidifier unit 2 to a point close to the patient). The inspiratory conduit 16 is therefore required to have a wall that is strong enough to support its own weight without collapsing. As the inspiratory conduit 16 is typically relatively long (e.g. 8 to 10 feet), this additional length and the thicker wall structure adds to the weight of the inspiratory conduit 16. If the outlet of the inspiratory conduit 16 is connected directly to the patient interface in such a manner that the patient is required to support this weight, this can cause discomfort to the patient due to the weight of inspiratory conduit 16 acting on the patient. Furthermore, the weight of the inspiratory conduit 16 can pull on the patient interface 17 and cause it to become dislodged or misaligned. A lighter, shorter secondary conduit (e.g. gases inlet conduit 62) running between the outlet of the inspiratory conduit 16 and the patient interface 17 can be used.

Gases inlet conduit 62 is lighter and shorter than the inspiratory conduit 16, and as outlined above, is generally used with e.g. a lanyard 63 connected to the gases inlet conduit 62 or to the connection between the inspiratory conduit 16 and the gases inlet conduit 62. In use, the lanyard 63 (as outlined above) supports at least a portion of the weight of the inspiratory conduit 16, such that the patient interface 17 only needs to support the comparatively lighter the gases inlet conduit 62. Furthermore, in configurations in which the lanyard 63 connects to the end of the gases inlet conduit 62, the patient does not need to remove the lanyard 63 when disconnecting the inlet conduit 62 from the inspiratory conduit.

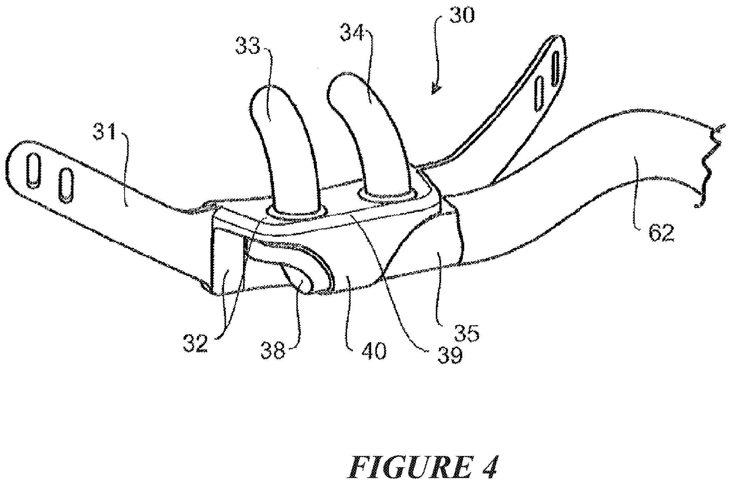

Various aspects of the nasal cannula 30 shall now be described in more detail with reference to Figures 4 to 33. Unless otherwise stated, the nasal cannula 30 illustrated in Figures 4 to 33 includes all of the features of the generalized nasal cannula described with reference to Figure 3.

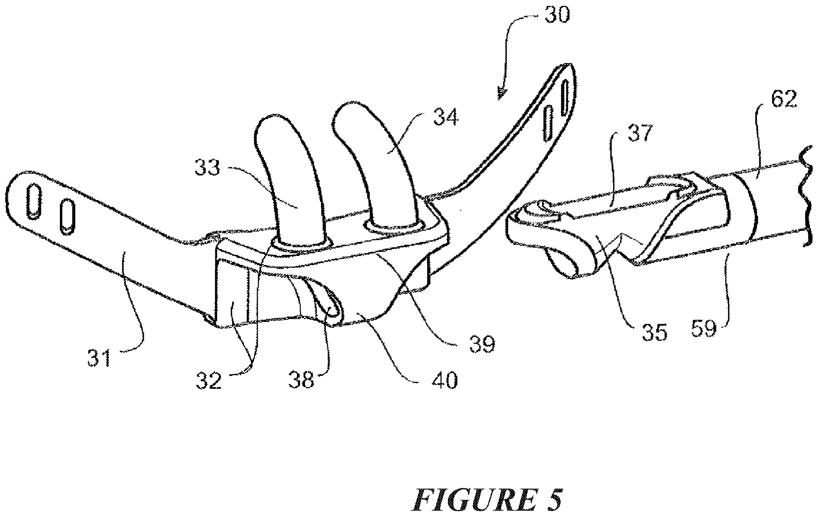

The nasal cannula 30 comprises two main parts: an interface connector 35 and a body 32. Example configurations of these two parts will now be described with particular reference to Figures 4 and 5.

The interface connector 35 is in use connected to and in fluid communication with the gases inlet conduit 62 as has been described above. However, it may be connected directly to the inspiratory conduit 16 in alternative embodiments.

The configuration of Figure 5 shows the interface connector 35 as being detachable from the remainder of the nasal cannula 30. Alternatively, the interface connector 35 may be an integral part of the nasal cannula 30. Alternatively, the interface connector 35 and the nasal cannula 30 form a one time fit, such that the user is prevented from disassembling the two components following the initial assembly. In the integrated or one time fit configurations, a continuous gases flow path is formed through the inspiratory conduit 16, the gases inlet conduit 62, the interface connector 35, and to the prongs of the nasal cannula 30.

In some configurations interface connector 35 is generally tubular in shape having a substantially circular inlet 59 on one side that curves to an oval or elliptical outlet 37, the outlet 37 being formed on one side of the interface connector 35 so that it is perpendicular to the inlet 59. The circular inlet 59 in the illustrated form receives the patient end of the gases inlet conduit 62, such that the gases flow from the gases inlet conduit 62 can pass through the interface connector 35.

In some configurations the interface connector 35 is integrated with or permanently coupled to the gases inlet conduit 62. Alternatively, the interface connector 35 is removably attached to the gases inlet conduit 62. The interface connector 35 engages with the body 32 so that the gases flow can pass through the outlet 37 and transfer from the gases inlet conduit 62 to the patient through the nasal prongs 33, 34 (described in detail below).

In some configurations the interface connector 35 is manufactured from a hard plastic material that only deforms under relatively high loading conditions (that is, it cannot easily be crushed in the hand of a user). The interface connector 35 may be moulded, injection moulded, machined or cast.

The interface connector 35 in use is connected to the body 32, so that the gases flow exiting the interface connector 35 enter the body 32. The body 32 will now be described in detail.

The body 32 includes the nasal prongs 33, 34 extending from a base portion 39 of the body 32. The gases flow passes through the body 32 to the nasal prongs 33, 34 and is delivered to the patient. In some configurations, the nasal prongs 33, 34 extend parallel to each other. In some configurations, the nasal prongs 33, 34 curve rearwards from the face mount portion 32. In some configurations, the nasal prongs 33, 34 curve towards each other. The structure of the prongs 33, 34 will be described in detail below.

The body 32 of the illustrated embodiment comprises side arms 31 and a tubular member 38 comprising a recess, integrally moulded together as shown in Figures 4 and 5. The tubular member 38 extends below the body 32 and is adapted to receive the interface connector 35 (for the configurations where the body 32 and the interface connector 35 are separable or separate items). The body 32 has a lip 39 that extends around the upper edge of the tubular member 38. The interface connector 35 is connected to the body 32 by a friction fit and the lip 39 on the body 32 helps to grip the interface connector 35 and form a sealed connection between the interface connector 35 and the body 32. The tubular member 38 comprises a rib 40 which extends below the body 32. The rib 40 helps to cradle and hold the interface connector 35 in the correct position as it engages with the body 32, the rib 40 extending around the outside of the interface connector 35. Outlet 37 on the interface connector 35 aligns in use with the underside of the face mount 32 portion when the interface connector 35 is connected to the body 32. This alignment reduces the amount of gases which leak out of the nasal cannula 30, allowing effective treatment of the user by delivering maximum amount of humidified gases.

The side arms 31 are used to attach the front straps 50 to the body 32. The side arms 31 extend from either side of the body 32. In some configurations, the side arms 31 are formed as an integral part of the body 32. In use, the front straps 50 are attached to the side arms 31 so that the patient interface can be worn by a user. In some configurations the ends of the front straps 50 are looped through a pair of slits on the side arms 31, with the ends including a hook and loop fastener or similar to hold the ends in place when they are looped back on themselves. Alternatively, the front straps 50 or loops 66 may be clipped onto the side arms 31, for example by way of co-operating male-female clips, or adhesively attached to the side arms 31.

In some configurations, the body 32, nasal prongs 33, 34, side arms 31 and the tubular member 38 are all manufactured as one continuous item. The body 32, nasal prongs 33, 34, side arms 31 and the tubular member 38 are all manufactured out of flexible polymer material such as a soft thermoplastic elastomer (TPE), or silicone.

The following is a description of the nasal prongs. In the following description the term "rear", or "back" or any such synonym refers to that part of the structure that faces towards and is closest to the patient's face when the nasal cannula is in use. The term "front" or "forward" or any such synonym refers to the side, face or part which faces away from and is furthest away from the face of a user of patient in use. The term "top" or "upper" refers to the side, face or part that is pointing away from the floor, when a user or patient wearing the interface is standing or sitting upright and looking forward. The term "bottom" or "lower" refers to the side, face or part that is directed or pointing toward the ground, again when a user or patient wearing the interface is standing or sitting upright and looking forward. For example, Figure 3 illustrates the patient interface 17 being worn by the patient, wherein the directions described above can be evaluated with reference to this figure. The definitions for these directions remain consistent throughout, including in figures where the patient interface 17 is shown without the patient.

In some configurations the body 32 includes two nasal prongs 33, 34 extending upwards and curving inwards from the upper surface of the body 32 as shown in Figures 4 to 10. Referring to Figures 4 to 10, the nasal prongs 33, 34 extend from the upper surface of the body 32 and each prong is placed in each nostril of the patient when the nasal cannula is in use. The prongs 33, 34 are configured to deliver the gases flow to a patient. The prongs 33, 34 receive the humidified gases flow from the gases inlet conduit 62 via the gases inlet conduit 62, the interface connector 35 and the body 32. The nasal prongs 33, 34 are therefore in fluid connection with the interface connector 35 and receive the gases flow from the gases inlet conduit 62.

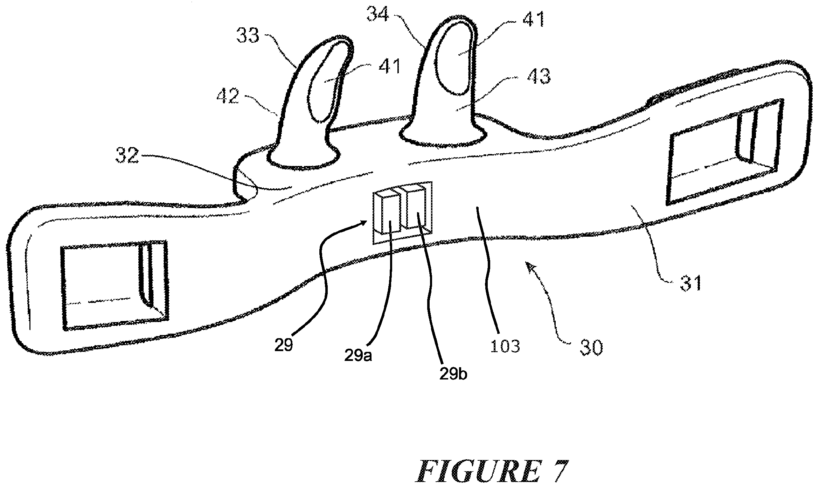

Referring to Figures 7 to 8 and Figure 12, a patient sensor 29 is located on the body 32 of the nasal cannula 30. In some configurations, the patient sensor 29 is located on the nasal cannula 30 such that it contacts the patient's skin during use. The patient sensor 29 may have an adhesive surface such that it can be secured in contact with the patient's skin.

The outer surfaces of the body 32 of the nasal cannula 30 may generally be divided into outwardly facing surfaces and inwardly facing surfaces. The term "outwardly facing surface" used herein can refer to an outer surface of the body 32 that faces away from the patient whilst the nasal cannula 30 is in use. The term "inwardly facing surface" used herein can refer to an outer surface of the body 32 that faces towards from the patient whilst the nasal cannula 30 is in use. The front and bottom sides of the body 32 can be considered to be outwardly facing surfaces, whilst the rear side can be considered to be an inwardly facing surface. The central portion of the top side of the body 32 that is located under the patient's nose in use may be considered to be an inwardly facing surface, whilst the remaining side portions of the of the top side may be considered to be outwardly facing surfaces.

Referring now to Figure 7, a first location for the patient sensor 29 is shown. In this configuration the patient sensor 29 is located on the rear surface 103 of the body 32. In the illustrated configuration the patient sensor 29 is located in the centre of the rear surface 103. Alternatively, the patient sensor 29 may be located anywhere on the rear surface 103 (e.g. rear surface is the surface closest to or in contact with the patient when the interface 17 is in use), such as along one of the two side arms 31. Providing the patient sensor 29 on the rear surface 103 causes the patient sensor 29 to be in contact with the patient's upper lip whilst the cannula is being worn.

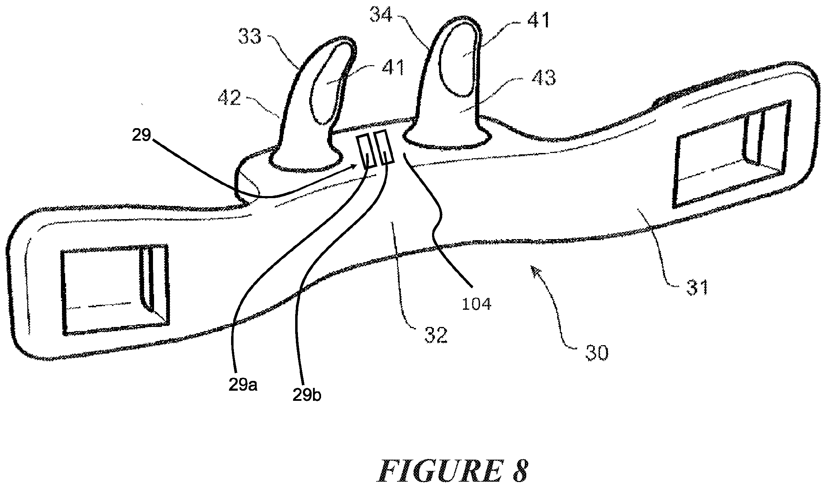

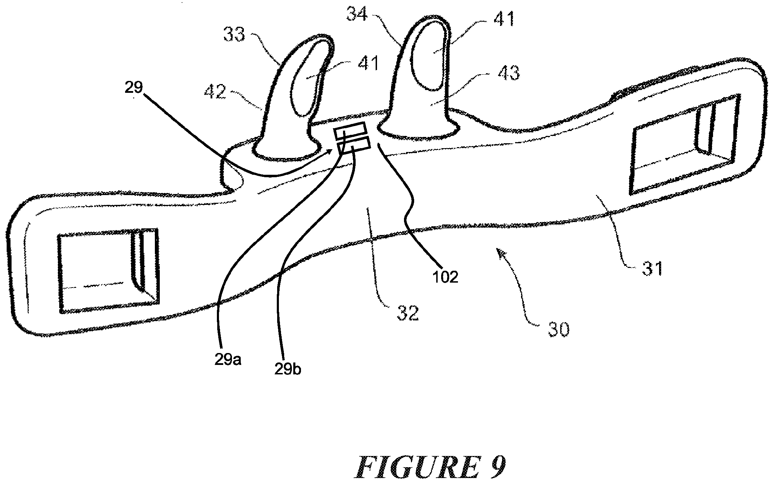

Referring now to Figures 8 and 9, a second location for the patient sensor 29 is shown. In this configuration the patient sensor 29 is located on the top surface 104 of the body 32, between the nasal prongs 33, 34. Providing the patient sensor 29 to this surface causes the patient sensor 29 to be in contact with the patient's columella whilst the cannula is being worn.

In some configurations, the patient sensor 29 is a pulse oximeter.

In the configurations illustrated in Figures 7 to 9, the patient sensor 29 is a reflectance pulse oximeter. The reflectance pulse oximeter comprises an emitter 29a and a receiver 29b. During use, the emitter 29a emits light which is reflected off of the patient's skin, and then received by the receiver 29b. Based on the wavelength of the received light, physiological parameters such as the patient's SpO₂ and heart rate can be calculated. The positions shown for the emitter 29a and the receiver 29b may equally be reversed. Furthermore, for the first location, the emitter 29a and the receiver 29b are shown to be spaced horizontally. In an alternative configuration, the emitter 29a and the receiver 29b are spaced vertically. In an alternative configuration shown in Figure 8, the emitter 29a and the receiver 29b are spaced horizontally in the second location. In an alternative configuration shown in Figure 9, the emitter 29a and the receiver 29b are spaced in front/behind each other in the second location. Further orientations may be equally applicable.

In some configurations of the nasal cannula 30, the patient sensor 29 may be located in a recess of the body 32 of the nasal cannula 30. The shape of the recess corresponds to the shape of the patient sensor 29, such that an outwardly facing surface of the patient sensor 29 sits flush with the surface of the body 32. In configurations where the patient sensor is a pulse oximeter, the outwardly facing surface of the patient sensor 29 is the operative surface of the patient sensor 29, i.e. the emitter 29a and the receiver 29b.

The patient sensor 29 can be considered to be "sitting flush" with a surface of the body 32 when the outwardly facing surface of the patient sensor 29 and the adjacent portions of the surface of the body 32 form a smooth combined surface. The combined surface is considered smooth when there is no significant indentation or protrusion at the boundary between the patient sensor 29 and the body 32. An indentation or protrusion is only considered significant if it is perceptible to the user by sight and/or touch.

In some configurations, the combined surface is flat. In other configurations, the combined surface is curved. In other configurations, the combined surface is a mixture of curved and flat portions. In some configurations, the outwardly facing surface of the patient sensor 29 is tangential to the adjacent portions of the surface of the body 32.

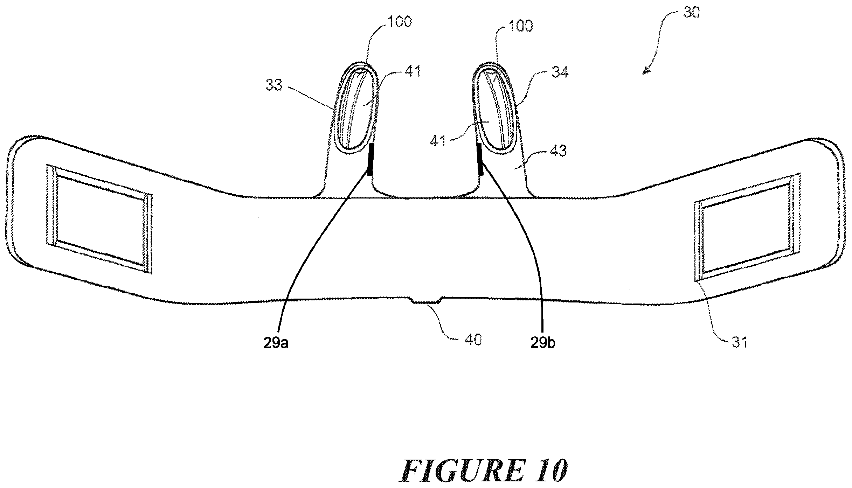

Referring now to Figure 10, a further configuration for the patient sensor 29 is shown. In this configuration the patient sensor 29 is located on the outer surface of one or more of the nasal prongs 33, 34. In a configuration in which the patient sensor 29 is a pulse oximeter, a transmissive pulse oximeter is used. The transmissive pulse oximeter comprises an emitter 29a and a receiver 29b. During use, the emitter 29a emits light which is transmitted through a portion of the patient's body, and then received by the receiver 29b. Based on the wavelength of the received light, physiological parameters such as the patient's SpO₂ and heart rate can be calculated. In some configurations, the emitter 29a is located on a centrally facing outer surface of one of the nasal prongs 33, 34, and the receiver 29b is located on the opposing centrally facing outer surface of the other one of the nasal prongs 33, 34, such that light is transmitted through the patient's septum.

Each of the nasal prongs 33,34 can have a notional central axis that runs through the centre of the lumen of each of the nasal prongs 33, 34 from the base to the tip. During use, the central axis is parallel to the direction of the gases flow. The nasal prongs 33, 34 may have a cross-section that has at least one flat edge, when said cross-section is taken perpendicular to the central axis defined above. For example, the cross-section may be a shape with entirely flat edges, such as a rectangle or a triangle. Alternatively, the cross-section may be a shape with a mix of one or more curved edges and at least one flat edge, such as a semicircle. This flat edge results in a flat surface along one face of each nasal prong 33, 34. In some configurations, the cross-section is consistent throughout the length of each nasal prong 33, 34. In other configurations, the size and/or dimensions of the cross-section changes throughout the length of each nasal prong 33, 34. For example, each nasal prong 33, 34 may taper inwards along its length, but maintain a semicircular cross-section throughout. In further configurations, the nasal prongs 33, 34 do not have a consistent cross-sectional shape throughout their length, but do have at least one flat outer surface.

The patient sensors 29 are located on the flat outer surface of the nasal prongs 33, 34. The flat surfaces of the nasal prongs 33, 34 may be located on the inner faces of the nasal prongs 33, 34 such that the two surfaces face each other. Using a flat surface as the location of the patient sensors 29 allows for a more consistent orientation, as a slight shift in placement during manufacturing does not result in a change in orientation. This is particular useful in the case of a transmissive pulse oximeter, as the transmissive pulse oximeters rely on the emitter 29a and the receiver 29b to be properly aligned. In some configurations, placing the patient sensors 29 on a flat surface aids in facilitating contact between the patient sensors and the patient's septum.

The nasal prongs 33, 34 may be inclined towards each other when not in use. Once the nasal cannula 30 is mounted on the patient the nasal prongs 33, 34 can elastically deform to fit the patient's nasal passages. This deformation results in a clamping force on the patient's septum. This clamping force helps to provide consistent contact between the patient's septum and the patient sensors 29.

Alternatively, a transmissive pulse oximeter may be set up to transmit through the outer nasal wall of the patient. In this configuration, either the emitter 29a or the receiver 29b is located on an outwardly facing outer surface of one of the nasal prongs 33, 34, whilst the other component of the emitter 29a and the receiver 29b is located on an additional projection that clamps onto the outer surface of the patient's nasal passage during use. In some configurations the additional projection extends from the body 32 of the nasal cannula 30. In some configurations the additional projection has a flat outer surface parallel to a flat outer surface on one of the nasal prongs 33, 34. The flat outer surface of the nasal prong 33, 34 faces towards the additional projection. One of each of the emitter 29a and the receiver 29b are located on one of each of the flat surfaces.

In a further alternative configuration, a reflective pulse oximeter may be used instead of a transmissive pulse oximeter. In this configuration, both the emitter 29a and the receiver 29b is located on the same nasal prong 33, 34. Alternatively, a reflective pulse oximeter may be used by including both the emitter 29a and the receiver 29b on a supplementary projection that contacts the outer surface of the patient's nasal wall. In some configurations the supplementary projection extends from the body 32 of the nasal cannula 30.

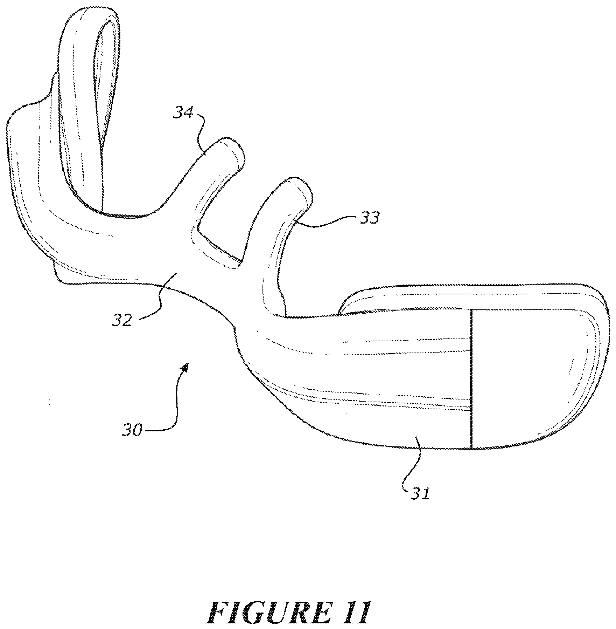

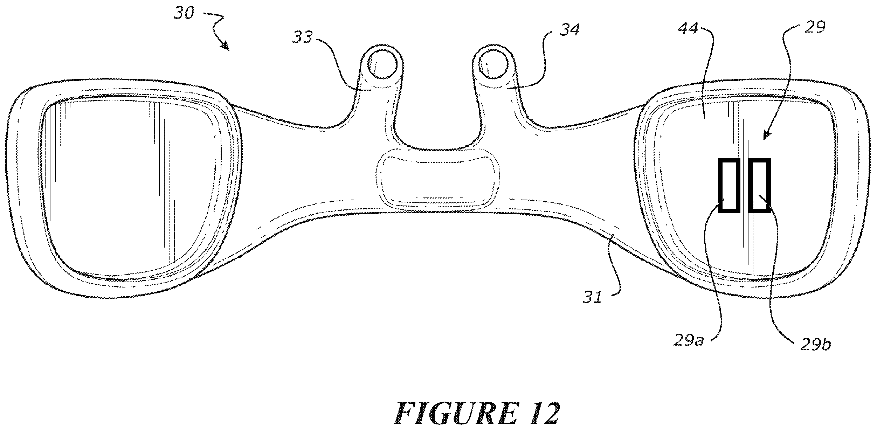

An alternative configuration of the nasal cannula 30 will now be described with referral to Figures 11 and 12. In this configuration the head securement assembly comprises one or more facial pads 44 located on the side arms 31. During use, the facial pads 44 may be attached to the patient's cheeks. In some configurations, the facial pads 44 have an adhesive surface that allows the facial pads 44 to be attached to the patient's cheeks. In certain scenarios this may be more comfortable for the patient, and may reduce the chance of the nasal cannula 30 shifting from its correct position during use. In order to allow the patient sensor 29 to contact the patient's skin, one or more cutouts may exist in the facial pads 44 corresponding to the position of the patient sensor 29.

In the configuration shown in Figure 12, the patient sensors 29 are located on a side arm 31 of the body 32 of the nasal cannula 30 in the same region as the facial pad 44. As the facial pad 44 is in contact with the patient's skin, a reflectance pulse oximeter (as described above) may be implemented. The emitter 29a and receiver 29b is located side by side in either facial pad 44. The emitter 29a and receiver 29b sits flush with the surface of the facial pad 44.

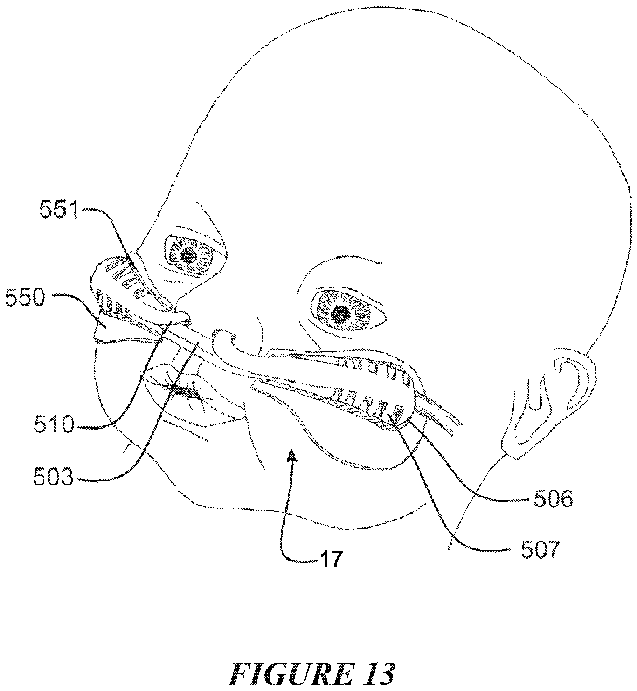

In an alternative configuration shown in Figures 13 to 15, the facial pads may comprise a two-part releasable attachment or connection arrangement 551. The releasable connection arrangement 551 acts between, and releasably connects, a pair of patches that are affixed to the patient and the patient interface 17 respectively.

The first patch is a dermal patch 550 that is adhered or otherwise attached to the patient's skin. The dermal patch has a patient side that faces the patient's skin and an interface side that faces the patient interface 17. The patient side of the dermal patch 550 may be attached to the skin of a patient by a dermatologically sensitive adhesive, such as a hydrocolloid. The patient interface side of the dermal patch is provided with the first part 553 of the two-part releasable attachment or connection system 551.

The second patch is a patient interface patch 552. The patient interface patch 552 also has a patient side and an interface side. The patient side of the patient interface patch 552 is disposed adjacent the dermal patch when the patient interface 17 is engaged. The complimentary second part of the two-part releasable attachment or connection system 553 is affixed to the patient side of the patient interface patch 552, so that the respective parts of the two-part releasable attachment or connection system 551 are easily engageable when the patches 550, 552 are brought together. The interface side of the patient interface patch 552 is affixed to the patient interface 17. The patient interface patch may be integrated with or suitably adhered to the patient interface 17.

A part or corner of the patient interface patch 552 may include a region that does not attach to the dermal patch 550. The general purpose of this is to allow a region (or tab) that can be more easily gripped by a patient for removing or detaching the patient interface 17 from the dermal patch. For example, the backing 2004 may also comprise of such a corner region.

The two-part releasable attachment or connection arrangement 551 may comprise a hook and loop material (such as Velcro^{™}), a magnet or an array of magnets disposed on the respective patches with the poles suitably arranged, an adhesive arrangement that is activated when the patches are urged together, or any other suitable releasable coupling. The interface side of the dermal patch 550 may have one of a hook or a loop material, and the patient side of the patient interface patch 552 may have the other of the hook or loop material, such that the dermal and patient interface patches are releasably attachable to each other.

In this configuration, the patient sensor 29 mentioned above is still located on a side arm 31 of the body 32 of the nasal cannula 30 in the same region as the facial pad.

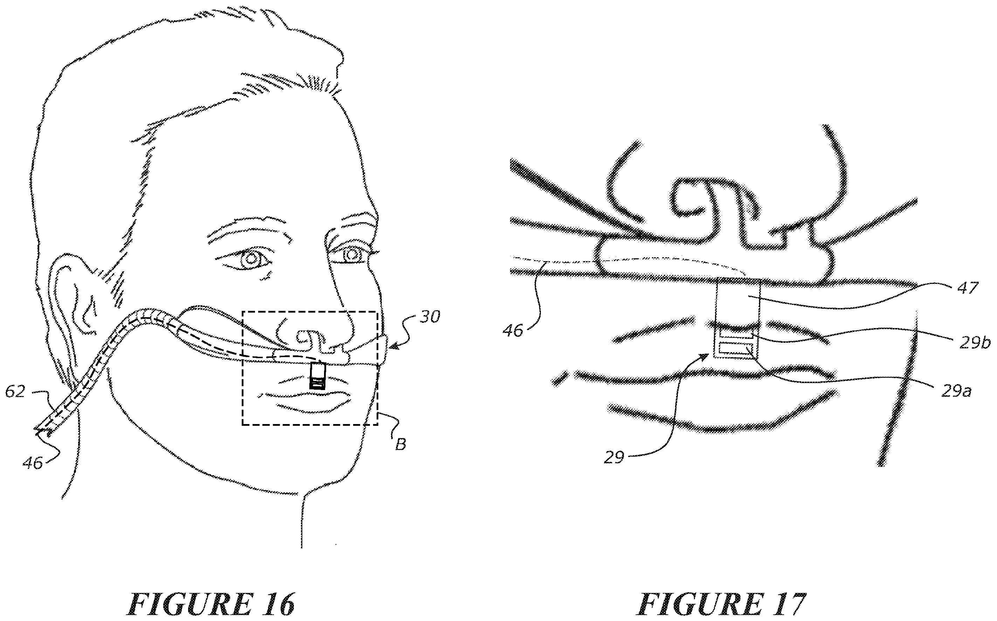

In order to provide power to and receive data from the patient sensors 29, one or more wires 46 connect the patient sensors 29 to the controller 13. The wires 46 may be attached to, or mounted on or in, the body 32, the interface connector 35, and/or the gases inlet conduit 62 of the nasal cannula 30, see for example Figures 16 to 20

There are a number of ways in which the wires 46 can be attached to, or mounted on or in, the body 32, the interface connector 35, and/or the gases inlet conduit 62, in accordance with this disclosure. For example, in some configurations, the wires 46 are embedded into the material of the body 32 and the gases inlet conduit 62 of the nasal cannula 30. In some configurations, the wires 46 are attached to the inner surface of the body 32, the interface connector 35 and the gases inlet conduit 62. In some configurations, the wires 46 are attached to the outer surface of the body 32, the interface connector 35 and the gases inlet conduit 62. In some configurations, a mesh wrap comprising interwoven filaments surrounds the body 32, the interface connector 35 and/or the gases inlet conduit 62. In some configurations at least a portion of the filaments are at least partially metal. In some configurations at least a portion of the filaments are at least partially plastic. In some configurations at least a portion of the filaments are at least partially made of a natural fibre. In some configurations, the wires 46 are interwoven with the filaments of the mesh wrap. Alternatively, any two or more of these configurations may be combined. For example, the wiring 46 may be embedded into the material of the body 32 and then attached to the outer surface of the interface connector 35 and the gases inlet conduit 62.

In configurations in which the interface connector 35 is removably attached to the body 32, the interface connector 35 and the body 32 each comprise one or more electrical contacts to allow the formation of an electrical connection between the wires 46 on the interface connector 35 and the body 32 when the nasal cannula 30 is assembled. The wires 46 in the interface connector 35 pass into the gases inlet conduit 65, with the opposing end of the gases inlet conduit 62 further comprising a connector comprising additional electrical contacts that correspond to electrical contacts on a connector of the inspiratory conduit 16. The electrical contacts of the gases inlet conduit 62 and the inspiratory conduit 16 may be configured such that an electrical connection is formed automatically if the two components are pneumatically connected. For example, the electrical contacts of the gases inlet conduit 62 and the inspiratory conduit 16 may be configured such that the pneumatic connection cannot be formed without the electrical connection also being formed. Additionally, the electrical contacts of the gases inlet conduit 62 and the inspiratory conduit 16 may be configured such that electrical connection cannot be formed without the pneumatic connection also being formed. The corresponding electrical contacts may be configured to contact each other when the pneumatic connection is formed. The corresponding electrical contacts may comprise planar surfaces. Additionally, or alternatively, the electrical contacts may comprise a pin and socket arrangement. This configuration offers the advantage of further reducing the setup time of the respiratory support system by not further requiring that a separate electrical connection be formed between the respiratory support apparatus 10 and the patient sensor 29.

As the electrical contacts of the gases inlet conduit 62 and the inspiratory conduit 16 may form an electrical connection automatically when the two components are connected pneumatically, the electrical contacts of the gases inlet conduit may non-moveable relative to the remainder of the connector.

In a further configuration, the patient interface 17 comprises one or more patient sensors 29 located on a sensor arm 47, which will now be described with reference to Figures 16 to 25. The sensor arm 47 may be relatively rigid, in that it cannot be easily deformed by the user. Alternatively, the sensor arm 47 may be resiliently deformable, in that it can be easily deformed by the user. The sensor arm 47 may have a patient contacting surface configured to contact the patient's skin. The patient contacting surface may be coated with an adhesive to ensure contact between the sensor arm 47 and the patient's skin.

The sensor arm 47 comprises a patient sensor 29 such as a pulse oximeter. The pulse oximeter may be a reflectance pulse oximeter comprising an emitter 29a and a receiver 29b as described above. Unless otherwise stated, the wiring 46 for the sensor arm 47 configurations is substantially the same as described above for the previous configurations without the sensor arm 47.

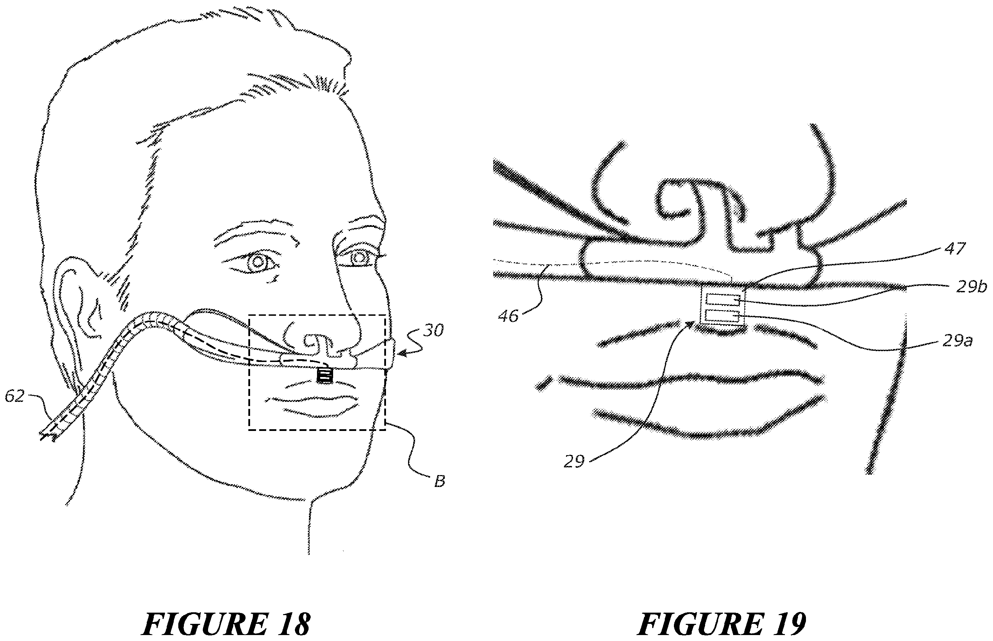

In the configurations shown in Figures 16 and 17, the sensor arm 47 is located on the lower side of the nasal cannula 30. The sensor arm 47 may be located near the centre of the nasal cannula 30. The position and dimensions of the sensor arm 47 are designed such that the patient sensors 29 contact the patient's upper lip whilst the nasal cannula 30 is in use. Alternatively, in the configurations shown in Figures 18 and 19, the position and dimensions of the sensor arm 47 are designed such that the patient sensors 29 contact the patient's skin above their upper lip whilst the nasal cannula 30 is in use.

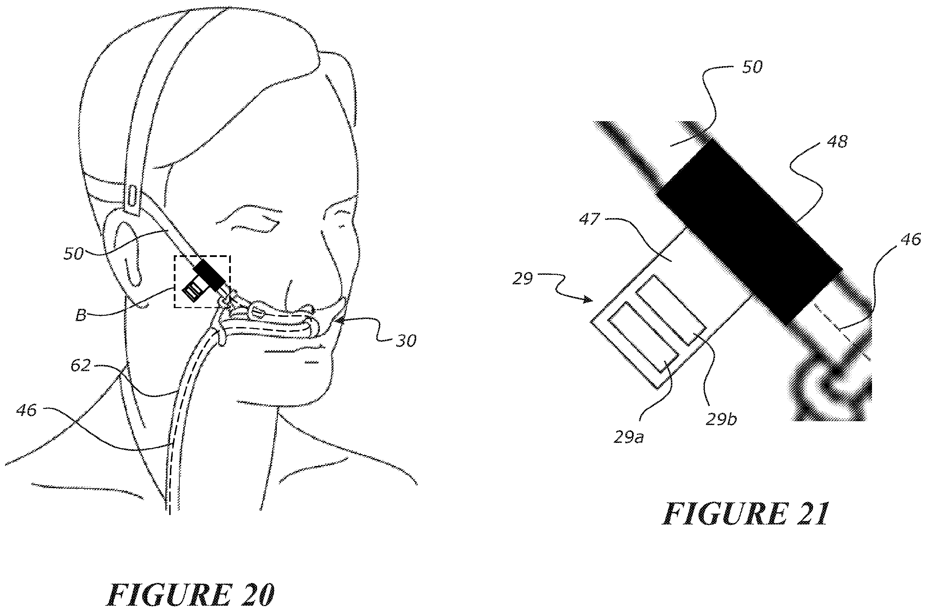

In a further configuration, the sensor arm 47 extends from a sensor mount 48, which will now be described in detail with reference to Figures 20 to 25. In the configurations shown in Figures 20 and 21, the nasal cannula 30 further comprises a sensor mount 48 located on a portion of one or more of the straps, such as one of the front straps 50. The sensor mount 48 may be fixedly attached to one of the front straps 50. Alternatively, the sensor mount 48 may be removably attached to one of the front straps 50. Additionally, or alternatively, the sensor mount 48 may be slidably attached to one of the front straps 50. Alternatively, the sensor mount 48 may be removably attached to the gases inlet conduit 62. Additionally, or alternatively, the sensor mount 48 may be slidably attached to the gases inlet conduit 62. The slidable pulse oximeter can be slid along the face to locate the sensor in an appropriate location on a face to get a pulse oximeter (i.e. SpO2) reading. For example the sensors may be located in a cheek region.

The sensor arm 47 extends from the sensor mount 48 and is configured to contact the patient's skin whilst the nasal cannula 30 is in use. The sensor arm 47 may be arranged such that it extends from the sensor mount 48 perpendicular to the front strap 50. In a configuration in which the sensor mount 48 is slidably mounted to one of the front straps 50, this direction is perpendicular to the direction of travel of the sensor mount 48.

The sensor arm 47 may be fixedly attached to the sensor mount 48.

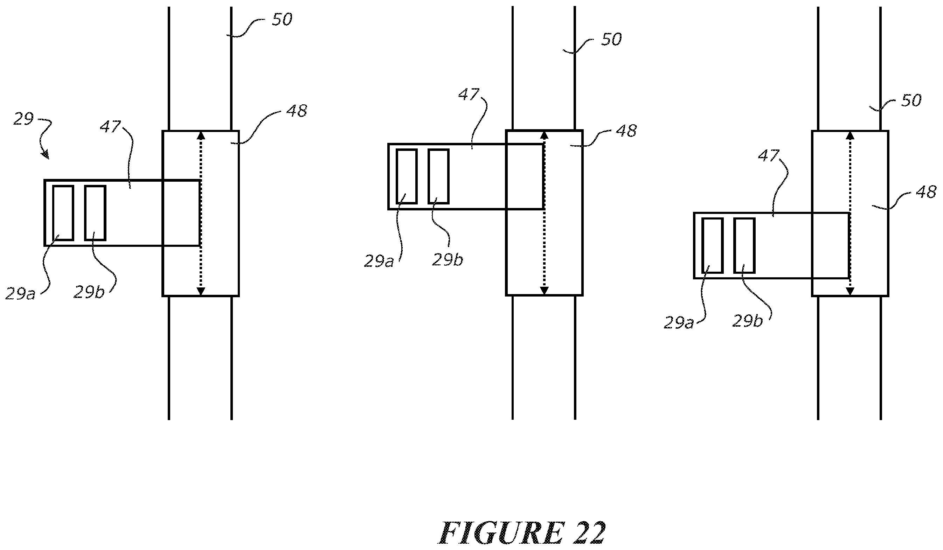

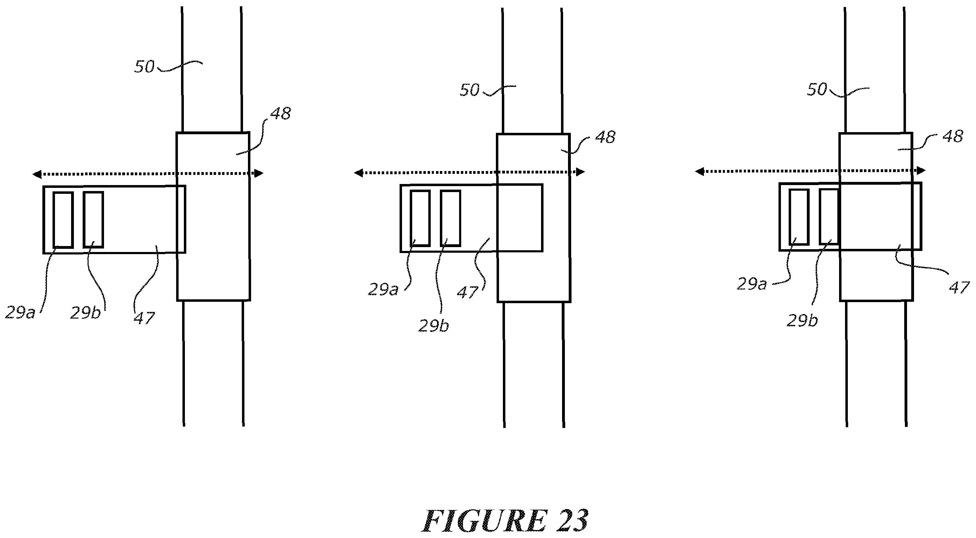

Alternatively, as shown in Figures 22 and 23, the sensor arm 47 may be slidably attached to the sensor mount 48. As shown in Figure 22, the sensor arm 47 may be able to slide in the same direction as which the front strap 50 extends. Additionally, or alternatively, the sensor arm 47 may be able to slide in a direction perpendicular to the direction in which the sensor arm 47 extends from the sensor mount 48. Additionally, or alternatively, as shown in Figure 23, the sensor arm 47 may be able to slide in a direction perpendicular to the direction on which the front strap 50 extends. Additionally, or alternatively, the sensor arm 47 may be able to slide in the same direction as which the sensor arm 47 extends from the sensor mount 48.

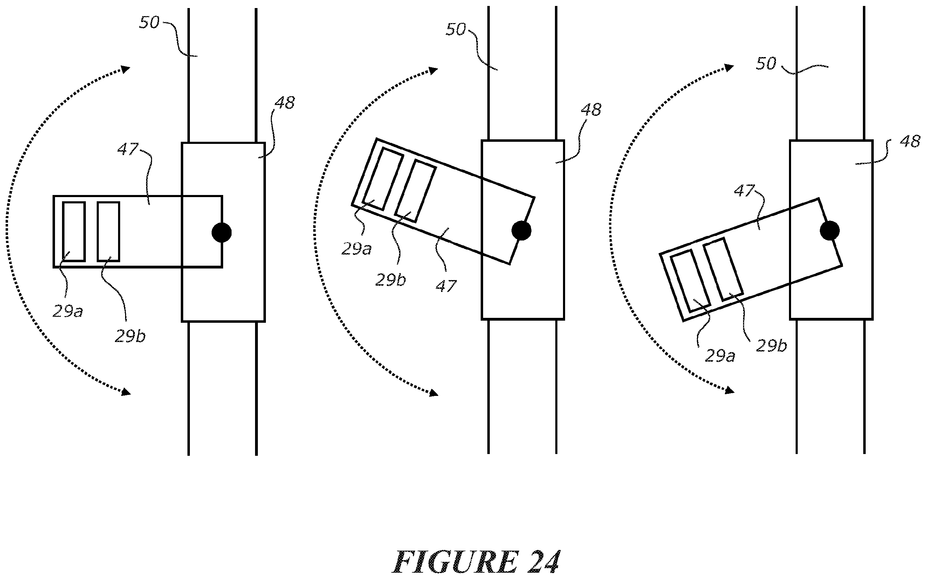

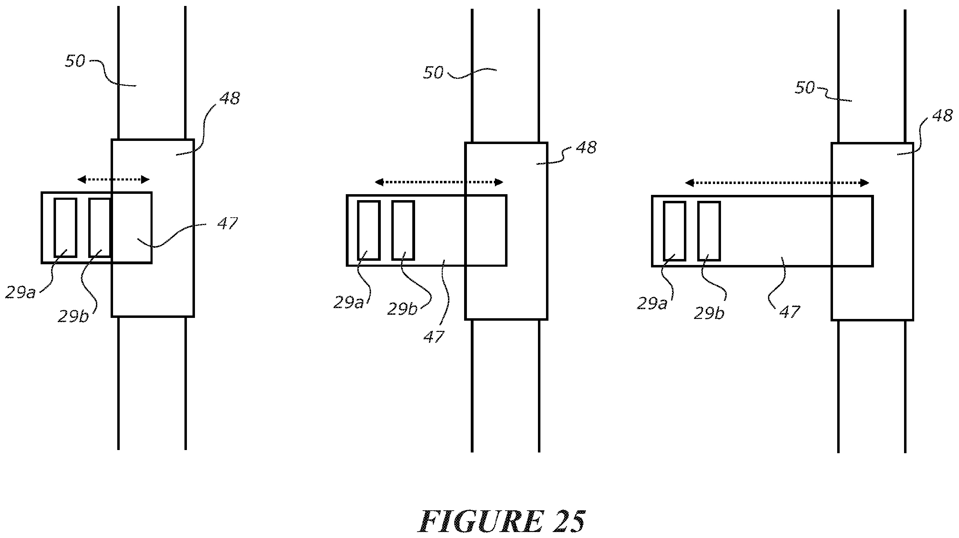

Additionally, or alternatively, as shown in Figure 24, the sensor arm 47 may be rotatably mounted to the sensor mount 48. Additionally, or alternatively, as shown in Figure 25, the sensor arm 47 may be able to extend and retract telescopically relative to the sensor mount 48.

By having the sensor mount 48 removably and/or slidably attached to one of the front straps 50, and/or having the sensor arm 47 movably attached to the sensor mount using one or more of the techniques described above, the user is able to adjust the position the of the patient sensor as needed, while still being able to set up the system relatively quickly. Additionally, the system allows the user to adjust the patient sensor as required while still benefitting from the integrated electrical and pneumatic connectors of the nasal cannula 30. The sensor and sensor mount is adjustable to allow the sensor to be located on the face of the patient to get an accurate SpO2 measurement.

In a further configuration, the sensor arm 47 is replaced by a sensor clip, thereby allowing the patient sensor 29 to be clipped onto a part of the patient, such as the ear lobe. In this configuration a transmissive pulse oximeter is used instead of a reflectance pulse oximeter.

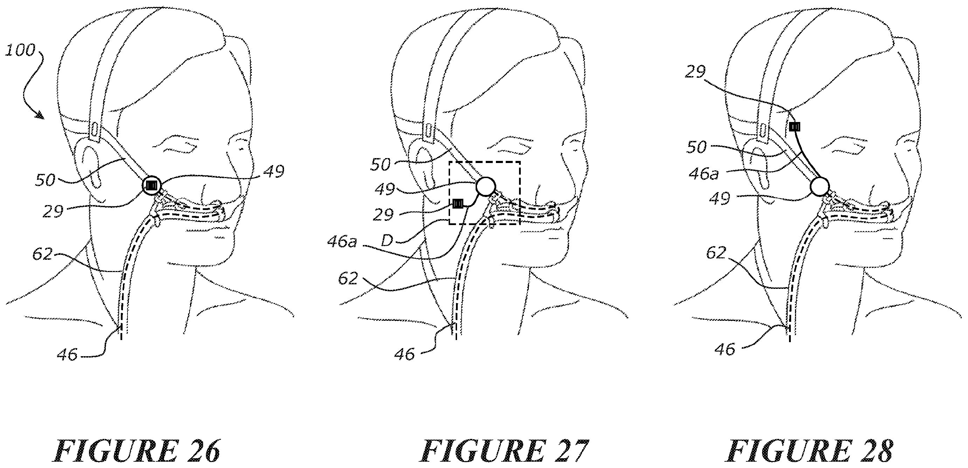

A further possible configuration involving a wire coil will now be described in relation to Figures 26 to 28. In this configuration, the sensor mount 48 is replaced by a wire coil 49. The wiring 46 leading up to the wire coil 49 is substantially the same as the previously described configuration.

As shown in Figures 26 to 29, the sensor arm 47 may be connected to the wire coil 49 by a secondary wire 46a. The sensor arm 47 may have any of the features described for the sensor arm 47 in the previous configuration. In this particular configuration, the sensor arm 47 may have an adhesive surface to allow it to adhere to the patient's skin. This arrangement of the sensor allows for the sensor 29 to be located in the temporal region of the face or on the frontal region of the forehead or on the mental region near the lower lip, where there are blood vessels that can be used to get blood oxygen (i.e. SpO2) readings. The structure of the wire coil provides adjustability of the sensor 29 position to a region on the fac where there are larger blood vessels useful for obtaining SpO2 readings.

As shown in Figure 26, the sensor arm 47 starts in an initial position, in which the sensor arm is located on or next to the wire coil 49, with the secondary wire 46a being substantially completely retracted into the wire coil 49. As shown in Figures 27 and 28, the sensor arm 47 can then be moved to a location on the patient's skin such that patient sensor 29 can begin measuring the one or more patient parameters. As the sensor arm 47 is moved onto the patient's skin, the secondary wire 46a uncoils as much as necessary from the wire coil 49. This arrangement provides the user with added flexibility in their placement of the patient sensor 29. For example, Figures 27 and 20C illustrate examples of how the patient sensor 29 can be placed on the patient's cheek or temple depending on the preference of the user and/or patient.

The secondary wire 46a is able to retract into the wire coil 49. The wire coil 49 may have a coil spring mechanism. A coil spring mechanism allows for compact storage of the secondary wire 46a without requiring much input by the user when the recoiling needs to be performed. The coil spring mechanism may be configured to automatically retract the secondary wire 46a. This is advantageous in that there is a reduced chance of the user forgetting to recoil the secondary wire 46a. Additionally, automatic retraction means that the only minimum required length of the secondary wire 46a is uncoiled, thereby reducing the chance that it becomes tangled with other components. Alternatively, coil spring mechanism may be configured to only retract the secondary wire 46a when the user actuates a component, such as a switch, button, lever, or the like. This is advantageous in that there is no tension in the secondary wire 46a during use, thereby potentially increasing patient comfort and reducing the chance of the sensor arm 47 becoming dislodged.

The wire coil 49 may be fixedly attached to one of the front straps 50. Alternatively, the wire coil 49 may be removably mounted to one of the front straps 50, such that it can removed and replaced on a separate section of one of the front straps 50 and/or on a sperate part of the nasal cannula 30 by use of a clip or any other suitable connector. To facilitate this, the wire coil 49 may have a second coil spring mechanism corresponding to a second wire that connects the wire coil 49 to the wire 46 of the nasal cannula 30. The second coil spring mechanism may have any of the same features as the first coil spring mechanism.

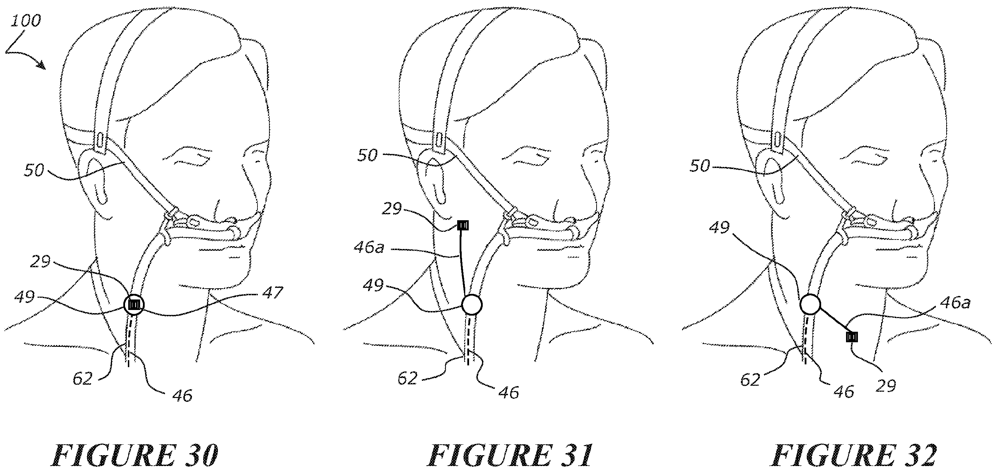

In a further configuration, the wire coil 49 may be fixedly or removably attached to the gases inlet conduit 62 as shown in Figures 30 to 32. In this configuration, the wire coil 49 may connect to the wire 46 as it passes through the gases inlet conduit 62. This configuration may be more suitable if the user wishes to attach the patient sensor 29 to the patient's neck or chest. The coil 49 allows for adjustability which assists in getting more accurate SpO2 readings by moving the sensor 29 in various locations.

The maximum length of the secondary wire 46a is set based on the expected maximum necessary length. The expected maximum necessary length depends on the expected locations at which the user might wish to place the patient sensor 29. For example, a longer maximum length may be provided in order allow user to attach the patient sensor 29 to the patient's chest, upper back, or shoulders. In one configuration the secondary wire 46a has a maximum length of about 300mm.

In a further configuration, the sensor arm 47 is replaced by a sensor clip, thereby allowing the patient sensor 29 to be clipped onto a part of the patient, such as the ear lobe. In this configuration a transmissive pulse oximeter is used instead of a reflectance pulse oximeter. This variation may be applied to any of the wire coil configurations described above.

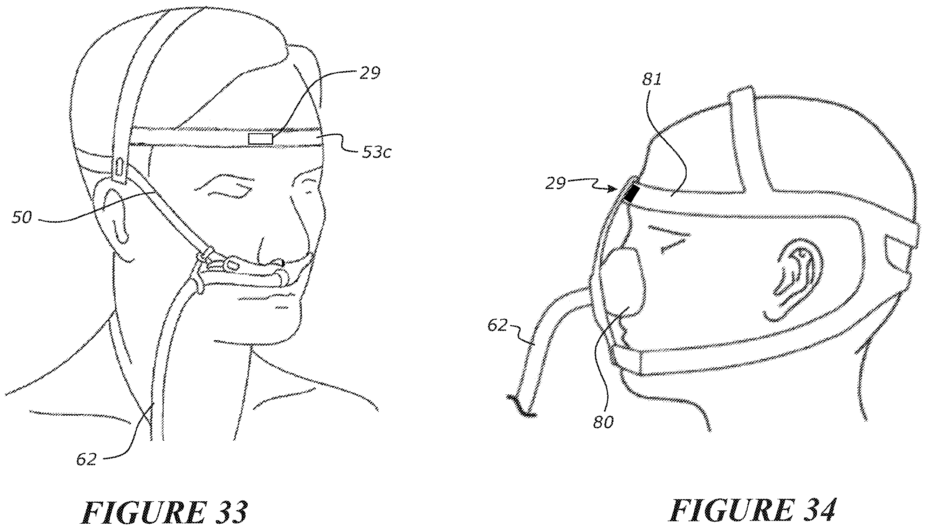

A further configuration in which the patient sensor 29 is integrated into the head securement assembly will now be described with reference to Figures 33 to 36.

Referring now to Figures 33, a configuration is shown in which a patient sensor 29 is incorporated into a forehead strap 53c. The patient sensor 29 may be a reflectance type pulse oximeter as previously described. The forehead strap 53c may be made of an elastic material, thereby helping to stabilize the head securement assembly as well as to maintain contact between the patient sensor 29 and the patient's forehead. In this configuration the wire 46 continues from the side arms 31 into the head securement assembly to the location of the patient sensor 29. In configurations in which the side arms 31 are removably attached to the head securement assembly, the head securement assembly and side arms 31 comprise corresponding electrical contacts.

In the previous listed configurations, the patient interface 17 is a nasal cannula 30. Referring now to Figure 34, a configuration is shown in which the patient interface 17 is a sealed nasal mask 80. Alternatively, the patient interface may be another type of sealed interface, such as an oral mask, a full face mask, a nasal pillows interface, or the like. Sealed interfaces typically utilize a forehead support 81, in which the patient sensor 29 may be located. The patient sensor 29 may be a reflectance type pulse oximeter as previously described. In this configuration the wire 46 continues from the patient interface 17 into the head securement assembly to the location of the patient sensor 29. In configurations in which the patient interface 17 are removably attached to the head securement assembly, the head securement assembly and patient interface 17 comprise corresponding electrical contacts.

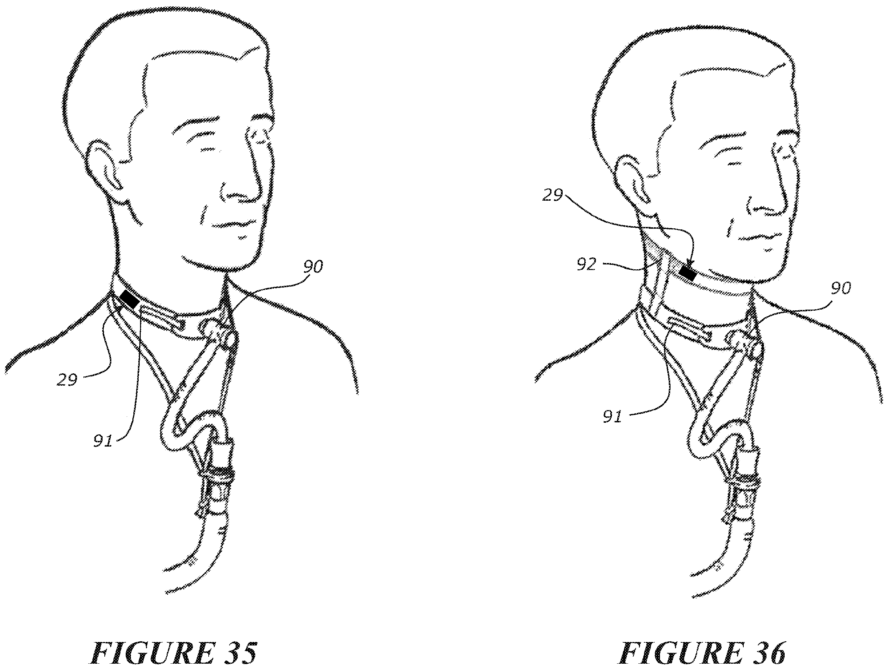

Referring now to Figures 35 and 36, configurations are shown in which the patient interface is a tracheostomy interface 90. As shown in Figure 35, the patient sensor 29 may be placed in a neck strap 91. Alternatively, the patient sensor 29 may be paced in a sensing neck band 92. The sensing neck band 92 may be made of an elastic material to promote contact between the patient sensor 29 and the patient's skin. The wiring 46 for the patient sensor 29 may be built into the neck strap 91 and/or the neck band 92, as described for the previous configurations. The patient sensor 29 may be a reflectance type pulse oximeter as previously described.

The above features of a patient sensor 29 may be used in conjunction with a nasal cannula substantially as described in our earlier international patent application WO2014/182179 filed 7 May 2014.

For example, a nasal cannula can be provided as described in WO2014/182179 comprising a body configured to engage with an orifice of the patient and direct the gases flow to said orifice. Such a nasal cannula can comprise part of a respiratory therapy system for example as described with reference to Figures 1 to 3. As described with reference to Figures 3 to 36, the nasal cannula can be provided with one or more sensors configured to measure a parameter. The one or more sensors are mounted (i.e. positioned) on the nasal cannula. Such embodiments are described in more detail below.

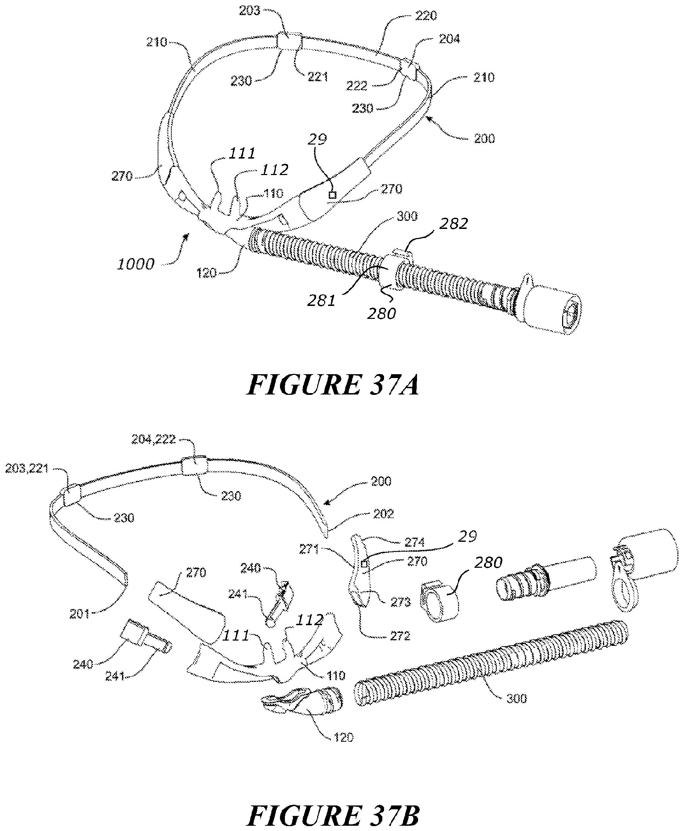

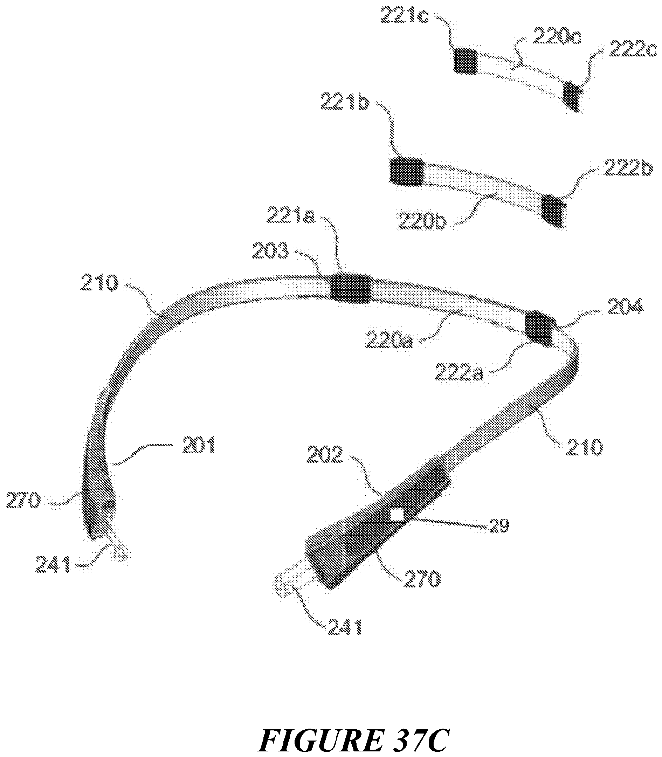

We refer now to Figures 37 to 43. These embodiments show a patient interface 101 configured to deliver breathing gases from a gases supply and humidification source (not shown) to the patient, and headgear 200 configured to support and retain the patient interface against the patient's face in use. The patient interface 101 is in the form of a nasal cannula 1000 that is adapted to couple an inspiratory conduit 300 and that comprises at least one, but preferably two, nasal prongs 111 and 112 configured to fit within the nares of a patient to deliver a flow of gases to the patient. The headgear 200 is in the form of a head strap 200 that is preferably adjustable in length to customise the size of the strap to the patient.

The nasal cannula 1000 comprises a face mount part 110 (i.e. body) including at least one, but preferably a pair of, tubular nasal prongs 111 and 112, integrally moulded with or removably attached to the face mount part 110 (i.e. body), and a gases flow manifold part 120 that is removably attached or integrally moulded to the conduit 300. The gases flow manifold part 120 is insertable into the face mount part from either one of two opposing horizontal directions, i.e. from either left side or the right side. In this manner, the position or location of the gases flow manifold part 120 is reversible with respect to the face mount part 110 (i.e. body). In other words, a user may choose to have the manifold part 120 (and essentially the conduit 300 extending there-from) extend from either the left side or the right side of the cannula 1000 depending on what is most convenient, for example depending on which side of the user the gas source or ventilator is located.

The face mount part 110 is formed from a soft and flexible material such as Silicone or other cannula material known in the art. The nasal prongs 111 and 112 are preferably supple and maybe formed from a sufficiently thin layer of Silicone to achieve this property.

The gases flow manifold part 120 is formed from a relatively harder material such as Polycarbonate, a High-Density Polyethylene (HDPE) or any other suitable plastics material known in the art. The face mount part 110 provides a soft interfacing component to the patient for comfortably delivering the flow of gases through the nasal prongs 111 and 112, while the gases flow manifold part 120 fluidly couples the conduit 300 to the nasal prongs 111 and 112 of the face mount part 110.

A patient sensor 29, such as a pulse oximeter or multiple pulse oximeter sensors, may be located on or in the manifold part 120.

The sensors 29 may be integrated into the manifold part 120 and therefore may be disposable. Alternatively, the sensors 29 may be removably mounted on the manifold part 120. The manifold part 120 may have an appropriate recess or receiving port/opening to receive the one or more sensor(s) 29. The one or more sensor(s) 29 may be removable and reusable.

The one or more sensor(s) 29 may be wireless or wired. The or one or more wires of the sensor(s) 29 can be routed through the manifold part 120, via the inlet 122 and back to the system controller via the inspiratory conduit 300.

The one or more sensor(s) are positioned on the manifold part 120 so as to position the sensor(s) 29 in contact with or adjacent the upper lip region e.g. in the oral region of the face. There are a number of blood vessels in the upper lip and the sensor(s) 29 can be used to determine blood oxygen via contact with or proximity to the upper lip region via the manifold part 120.

The manifold part 120 may be formed from a rigid plastics material as it is received into a soft silicone body of the cannula. The manifold part 120 being rigid makes it easier to insert the manifold part into the face mount portion and retain the manifold part in its operative position (i.e. inserted within the face mount). The manifold part is inserted into the face mount part and in fluid communication with the prongs to direct gases from the inlet conduit to the prongs. The sensors 29 located on or in the manifold part being positioned in the face mount part positions the sensor 29 in a sensing position i.e. the sensor is positioned adjacent or in contact with the upper lip.

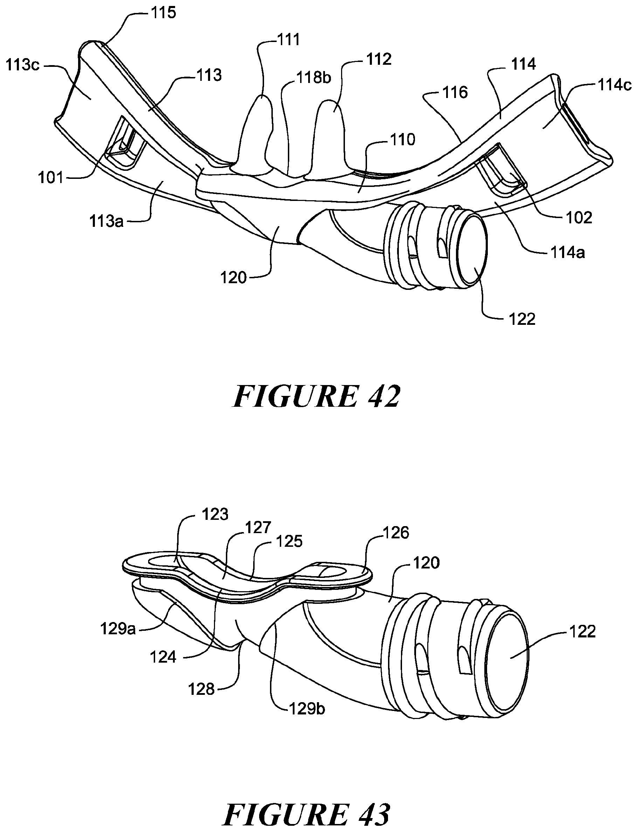

A patient's septum and/or columalla is generally quite a sensitive area and can be a source of discomfort when subjected to excessive contact pressure for prolonged periods. The nasal cannula of the present disclosure can alleviate or reduce this pressure by providing a cushioned region of the cannula 1000 adjacent the patient's septum/columalla. With reference to Figures 42 and 43, in an embodiment, the outlet 123 comprises a pair of opposed recesses or grooves 124/125 at the outer periphery for forming a dent or dip 127 in a region that locates adjacent the septum/columalla in use. When coupled to the face mount portion 110, this dip 127 creates a gap between the base portion 118 and the outlet 123 of the manifold 120. In use, the gap cushions/softens the region of the cannula 100 directly adjacent the septum/columalla. It disengages the pressure of the harder manifold part 120 from the septum/columalla and allows the septum/columalla to rest on the soft base of the face mount portion 110 only.

The base portion 118 is preferably also formed with a hollowed outer portion and/or dipped outer profile 118b between the prongs 111 and 112 to alleviate pressure at the septum/columalla. The hollowing should be as much as possible without (significantly) compromising the flow delivered to the patient. The dipped portion 118b is also preferably complementary to the periphery of the outlet 123 to maintain an effective seal between the two parts of the cannula.

The pulse oximeter 29 may be placed in between the prongs 111, 112 on the upper surface of the cannula such that it contacts the septum/columella. For example the pulse oximeter 29 is positioned within the dipped portion 118b between the prongs. The recessed manifold is advantageous when the sensor 29 is positioned between the prongs 111, 112 in order to reduce pressure sores or other pressure injuries due to the sensor 29 being in contact with the septum/columella. The recessed portion, that is, dip 127 in the face mount portion 110, allows the cannula to deform into the recessed section thereby ensuring sensor contact while alleviating pressure injury. The dip 127 may therefore be beneficial if the pulse oximeter 29 is located on the top surface of the base portion 118 (for example in a position as indicated in figure 9, between the two prongs 111, 112).

In the embodiment of Figures 37 to 41, the headgear used to retain the patient interface 100 against the patient's face comprises a head strap 200 of a single continuous length and adapted to extend in use along the patient's cheeks, above the ears and about the back of the head.

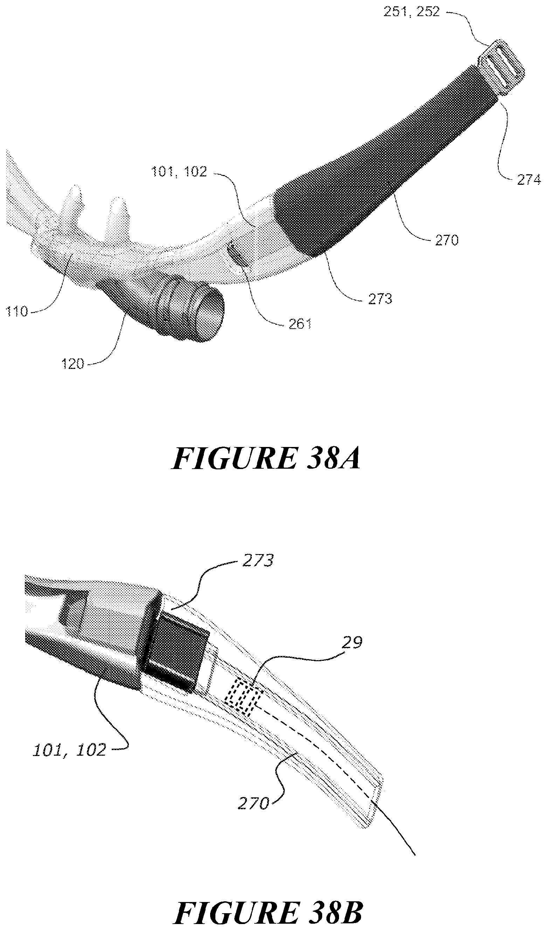

Primary end portions 201 and 202 of the strap 200 are adapted to releasably connect to respective formations 101 and 102 (see Figure 38A for example) on either side of the nasal cannula 100 to hold the cannula 100 in position during use.

A strap connector 230 is provided at each of the secondary end portions 203/204 of the main strap 210 and the respective end portions 203/204 of the strap segments 220.

Each connector 230 is provided with a strap connection mechanism at one end to couple to the strap material, and a coupling mechanism at an opposing end to releasably couple the respective end of a similar connector 230.

Cannula connectors 240 are provided at the primary end portions 201 and 202 of the main strap 210. These connectors 240 have a similar strap connection mechanism to the strap connectors 230 of the secondary end portions 203 and 204, but include a clip member, such as a push fit clip 241, at an end of the connector 240 opposing the strap ends. The clip 241 is configured to releasably couple the respective formation 101/102 at the side of the cannula 110. The clip member 241 is preferably a bendable part, such as a plastic part, that forms a hinged portion relative to the strap. The clip 241 is preferably preformed to have a curved shape along its length, such as one with an angle between flat and 20 degrees for example. This curve allows the clip 241 to fit the contour of the patient's face in the region of the clip 241.

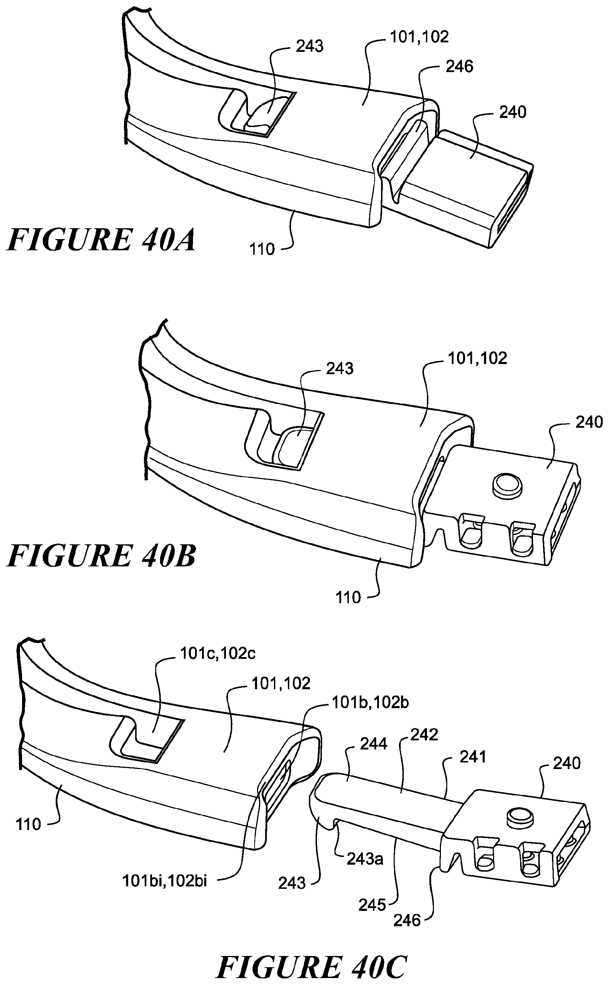

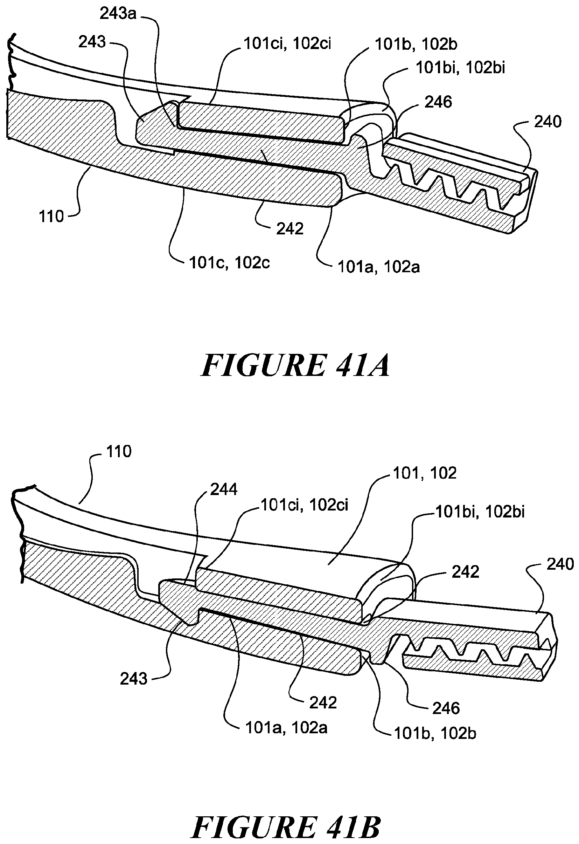

Referring to Figures 40 and 41, a method of engaging and disengaging each connector 240 of the head strap 200 to and from the patient interface 110 will now be described. Each connector 240 comprises a clip member 241 having an elongate connector body 242 and a lateral projection 243 at a terminal end of the body 242. The lateral projection 243 comprises an inwardly facing engagement surface 243a. The face 244 of the connector 240 opposing the face 245 from which the projection 243 extends is preferably substantially smooth or planar. The corresponding formation 101/102 of the cannula 110 comprises a channel 101a/102a having entry 101b/102b and exit 101c/102c apertures at either end of the channel 101a/102a. A peripheral wall of the exit aperture 101c/102c defines an abutment 101ci/102ci configured to engage with the surface 243a of the projection 243 of the clip member 241. A periphery 101bi/102bi of the entry aperture 101b/102b defines an abutment for engaging a flange 246 at an opposing end of the body 242 to the projection 243. This acts to limit the extent of insertion of the connector 240 into the corresponding channel 101a/102a. The flange 246 may be provided by a terminal end of the strap connection mechanism and/or the sleeve 270.

Each section on either side of the head strap 200 and adjacent the respective primary end portion 201/202 includes or has applied thereto a cheek support 270 comprising at least a surface region 271 for frictionally engaging with the user's face to stabilise the headgear 200 on the face at the cheek, such as the cheekbone or below or a region thereof, both during coupling of the headgear to the patient interface 100 and after when in use. The surface region 270 is preferably of a relatively higher frictional surface material than the remainder of the strap 200.

The high friction surface material 271 is adapted to extend over a portion of the side of a patient's face in use, preferably at or at least substantially towards the patient's cheek, to assist with retaining or stabilising of the patient interface 100 upon the face of a patient. The high friction surface material, being locatable at the cheek of the user, further assists in keeping a remainder of the head strap 200 separated from and preferably extending below the eye or the orbit of the eye of the user, so as to prevent obstruction of vision and/or discomfort resulting from the head strap 200 bridging at or near the eye or eye orbit.

It will be appreciated the high friction surface material 271 may be adapted to extend over a portion of the side of a patient's face in use, for example, extending from at or near or above the left and right outer upper lips rearwardly and upwardly across the left and right cheeks.

The frictional surface material may be provided in the form of an elongate sleeve 270 that is configured to receive the respective primary end portion 201/202 of the strap 200. The sleeve 270 is configured to removably couple (or alternatively be permanently coupled) about the strap 200, a section of the strap 200 and/or a cannula connector 240/260 at the primary end portion of the strap.

The sleeve 270 is coupled about the strap 210 at the primary end portion 201/202 and also about a portion of the connector 240. The strap 210 extends through a passage 272 in the sleeve 270, as can be seen in Figure 37B. The strap 210 is adapted to be threaded through this passage and preferably remains free to be stretched or elasticised or extended when in a sleeved configuration. The connector 240 is substantially housed by the sleeve 270 or shrouded by the surface region to minimise direct contact with the user's skin thereby improving stability comfort of the headgear 200. The clip 241 extends from an end 273 of the sleeve 270. In another embodiment, the sleeve 270 can be over-moulded on the connector 240 and/or the strap 210.

Referring to Figure 38A, the sleeve 270 may be coupled about the connector 260 extending from the strap 210 at the primary end portion 201/202. In this embodiment the connector 260 is substantially housed by the sleeve 270 or shrouded by the surface region to minimise direct contact with the user's skin thereby improving stability and comfort of the headgear 200. In other words, the connector 260 extends fully though the passage 272 of the sleeve 270. The buckle 251/252 extends from an end 274 of the sleeve 270 and the clip 261 extends from the opposing end 273.

The sleeve 270 may be pre-formed to have a curved shape along its length, such as one with an angle between flat and 20 degrees for example. The curve allows the sleeve 270 to fit the contour of the patient's face or cheek in the region of the sleeve in use. Alternatively the sleeve 270 may elastically or non-elastically deform to take on the shape of a curved sleeve upon engagement with the primary end portion 201/202 or connector 260 of the head strap 200.

The sleeve 270 provides a surface region 271 of relatively higher frictional surface material for frictionally engaging with the user's face or facial skin. This surface region 271 is to be positioned for frictional engagement with the facial cheek skin of a user. The surface region 271 is at least localised to the strap or the section of strap which is to be positioned upon the cheeks of a user. The surface region 271 provided with the relatively higher frictional surface material is preferably of a material that is smooth and comfortable on the skin of the patient. The sleeve 270 or at least the surface region 271 is therefore formed from a relatively softer material than the connectors 240 and 260.

In one preferred embodiment, the surface region 271 or the sleeve 270 is formed from a soft Thermoplastic Elastomer (TPE), but may alternatively be formed from another plastics material such as Silicone, or any other biocompatible materials.

Headgear for other forms of interface in addition to nasal cannula may comprise cheek supports 270 as described or similar, at or adjacent either side end of straps of headgear of the interface, which connect to the mask, for frictionally engaging with the user's face to stabilise the mask on the face at the cheeks, and particularly for example direct nasal masks comprising nozzles or pillows which enter or engage the nares of the wearer. Such headgear may again comprise a single head strap adapted to extend in use along the patient's cheeks, above the ears and about the back of the head, with ends comprising clips in any suitable form which couple to the mask on either side (or are permanently attached to the mask).

A patient sensor 29, for example in the form of a pulse oximeter, may be provided on the nasal cannula 1000 of Figures 37 to 41.

The patient sensor 29 may be provided on nasal cannula 1000 in accordance with any of the configurations described with reference to the nasal cannula 100 of Figures 1 to 36.

The patient sensor 29 may be provided on the headgear 200, or on another removable part of the nasal cannula 1000 that connects to the face mount part 110, or the gases flow manifold part 120 of the nasal cannula 1000. In this way, if the face mount part 110, and/or the gases flow manifold part 120 are replaced or disposed of, the patient sensor 29 can be retained with the headgear 200 or other removable part, so that the patient sensor 29 is not disposed of and can be reused. For example, the patient sensor 29 could be provided on headgear 200 which is configured to connect to multiple different sizes of face mount part 110, and/or gases flow manifold part 120. This allows a user to swap or replace parts of the cannula without having to dispose of the patient sensor 29.

Referring to Figures 37 to 41, the patient sensor 29 may be provided on sleeve 270. Any wiring associated with patient sensor 29 may extend through passage 272 of sleeve 270, and extend from the end 274.

Patient sensor 29 may be recessed into the face contacting surface 271 of the sleeve 270 and may be flush with that face contacting surface 271. Patient sensor 29 may be located in any suitable position along the length of sleeve 270, for example adjacent the formations 101, 102, or adjacent headgear strap 210.

Patient sensor 29 may be permanently mounted on the sleeve, for example the patient sensor 29 may be overmoulded onto the sleeve 270.

Patient sensor 29 may be removably mounted on the sleeve 270 so that the patient sensor 29 can be replaced, or reused if the sleeve 270 is disposed of. The patient sensor 29 can be removed, wiped and incorporated into a different cannula having a similar recess in the sleeve 270 to receive the sensor 29. This allows the sensor to be reused for patient's thereby reducing costs to a medical care facility.

Alternatively, the patient sensor 29 may be provided in a complimentary sensor body that can be permanently or removably mounted on the sleeve 270, for example in a corresponding recess on the sleeve 270. The recess and complimentary body may be provided with one or more retaining formations configured to retain the body in the recess. The sensor 29 being incorporated into the sleeve 270 provide the sensor 29 in contact with the cheek region e.g. the buccal or temporal regions of the face. There are blood vessels in this area of the face that the sensor can be positioned adjacent and used to detect the blood oxygen.

A patient interface, such as a nasal cannula in accordance with any of Figures 37 to 43, may comprise a plurality of patient sensors 29.

For example, the patient interface may comprise multiple sensors 29 (i.e. multiple pulse oximeters) incorporated into the patient interface. For example, each or at least one sleeve 270 may have one or a plurality of pulse oximeters 29 that are positioned on or in the side arm (i.e. sleeve 270). The measurements from these multiple sensors 29 can be averaged by the controller to provide a blood oxygen (SpO2) reading.

Each sleeve 270 may therefore comprise a single sensor 29.

In a further alternative form, each sleeve 270 (i.e. each side arm) of the cannula may comprise multiple sensors. One, some or all of the plurality of sensors may be removable. Each sleeve 270 may comprise a plurality of recesses or openings to receive sensors 29.

Multiple sensors 29 can be advantageous, as averaging the measured values can provide for a more accurate SpO2 reading and reduce noise in the sensor readings received by the controller.

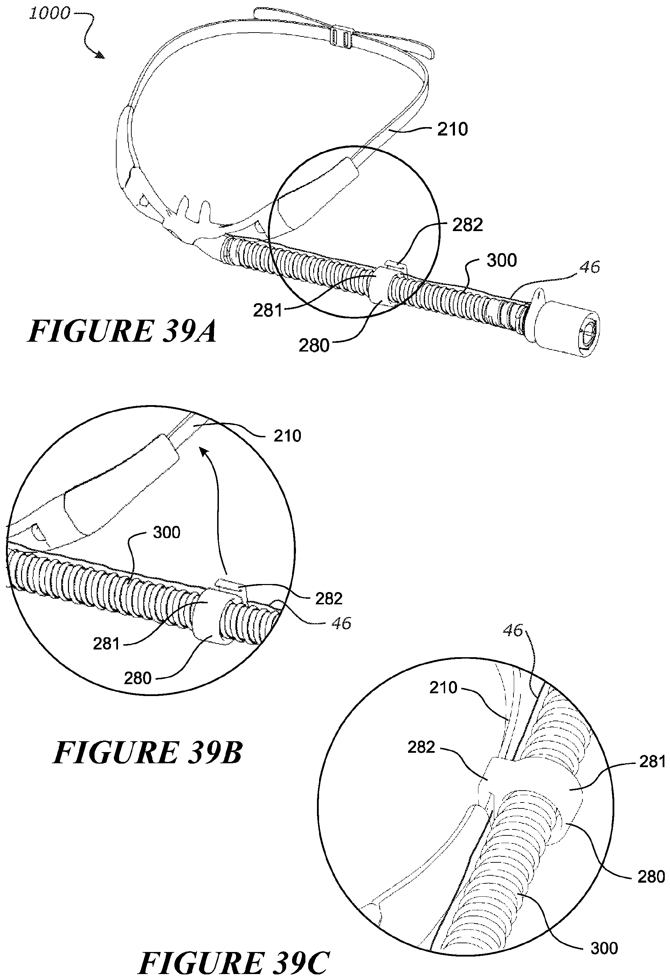

Referring to Figure 39, a retention clip 280 may be provided that comprises a tubular body 281 for receiving and accommodating a portion of the conduit 300 therein. A hook 282 projects from the body 281 to couple the strap or other component of the headgear 200. In this manner the conduit 300 can be coupled or tethered to the head strap 210 or headgear 200 in use. If the conduit 300 is pulled, the force will be exerted onto the head strap 210 and not directly on the cannula 100. This relocation of force will reduce the likelihood of the prongs 111 and 112 of the cannula 100 flicking out of the patient's nostrils.

One or more tethering points for connecting the clip 280 may be available on the headgear 200, with preferably at least two symmetric tethering points on either side of the headgear to increase usability.

It will also be appreciated the retention clip 280 may be removeable from or may be a permanent fitting on the gas supply tube 300.

The retention clip 280 may be connected or retained to a part of the patient interface, such as for example a part of an interface which provides for a relatively more rigid region (such as to facilitate support of the gas supply tube item 300).

The retention clip 280 may also be positioned or affixed at a particular location on the gas supply tube item 300, for example a predetermined location may be provided which holds the retention clip 280 in place.

The retention clip 280 may be configured to retain the wiring of the patient sensor 29, to secure the wiring against the conduit 300. The patient sensor wiring can therefore extend in parallel with the longitudinal axis of the conduit 300.

The conduit 300 may be provided with one or more sensor wires, for example in the wall of the conduit, or extending through the bore of the conduit. The one or more sensor wires may be configured to be electrically coupled to the patient sensor 29.

Such electrical coupling could be provided by a physical electrical coupling, such as via an electrical connector, between wiring of the patient sensor 29 and the one or more sensor wires in the conduit 300.

Such electrical coupling could be provided via an inductive coupling. For example patient sensor wiring may extend along sleeve 270 and/or may be provided in the face mount part 110, and/or the gases flow manifold part 120 of the cannula 1000. Conduit wiring may extend to a position at or adjacent an end of the conduit 300, where the conduit 300 is connected to the inlet of the nasal cannula. The conduit 300 and the cannula 1000 may be provided with inductive couplers configured to electrical couple the conduit 300 to the patient sensor wiring.

Such an arrangement would remove or reduce the need for a physical electrical connector or the like, and the need for one or more exposed electrical contacts. Such an arrangement would also remove or reduce the number of connections needing to be made by a user in use of the nasal cannula 1000. For example if the patient sensor 29 and headgear 200 were to be reused, the user would not have t physically disconnect the patient sensor wiring from the conduit 300.

The conduit 300 may be a heated or an unheated conduit. The conduit may be an extension of any desired length.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Where reference is used herein to directional terms such as 'up', 'down', 'forward', 'rearward', 'horizontal', 'vertical' etc., those terms refer to when the apparatus is in a typical in-use position, and are used to show and/or describe relative directions or orientations.

The terms "approximately," "about," and "substantially" as used herein represent an amount close to the stated amount that still performs a desired function or achieves a desired result. For example, in some embodiments, as the context may permit, the terms "approximately", "about", and "substantially" may refer to an amount that is within less than or equal to 10% of, within less than or equal to 5% of, and within less than or equal to 1% of the stated amount.

Reference to any prior art in this specification is not, and should not be taken as, an acknowledgement or any form of suggestion that that prior art forms part of the common general knowledge in the field of endeavour in any country in the world.

The disclosed apparatus and systems may also be said broadly to consist in the parts, elements and features referred to or indicated in the specification of the application, individually or collectively, in any or all combinations of two or more of said parts, elements or features.

Where, in the foregoing description reference has been made to integers or components having known equivalents thereof, those integers are herein incorporated as if individually set forth.

Depending on the embodiment, certain acts, events, or functions of any of the algorithms, methods, or processes described herein can be performed in a different sequence, can be added, merged, or left out altogether (for example, not all described acts or events are necessary for the practice of the algorithms). Moreover, in certain embodiments, acts or events can be performed concurrently, for example, through multi-threaded processing, interrupt processing, or multiple processors or processor cores or on other parallel architectures, rather than sequentially.

It should be noted that various changes and modifications to the presently described embodiments described herein will be apparent to those skilled in the art. Such changes and modifications may be made without departing from the scope of the disclosed apparatus and systems and without diminishing its attendant advantages. For instance, various components may be repositioned as desired. It is therefore intended that such changes and modifications be included within the scope of the disclosed apparatus and systems. Moreover, not all of the features, aspects and advantages are necessarily required to practice the disclosed apparatus and systems. Accordingly, the scope of the invention is intended to be defined only by the claims that follow.

## Claims

1. A nasal cannula interface (17) for supplying a gases flow to a patient comprising:
a gases inlet conduit (62) configured to receive the gases flow from a flow source via an inspiratory conduit (16), the gases inlet conduit (62) defining at least a portion of a gases flow path and comprising a patient end and a distal end,
a first set of wires (46),
one or more sensors (29) configured to be placed on the patient's skin and configured to measure at least one parameter, and
a nasal cannula (30) comprising
a body (32) having a base portion (39) and at least one prong (33,34) extending from the base portion (39), the at least one prong (33,34) being configured to direct the gases flow to an airway of the patient, and
an interface connector (35) for receiving the gases flow from the gases inlet conduit (62) and directing the gases flow towards the at least one prong (33,34), and wherein the patient end is connected to the interface connector (35),
the distal end comprises an interface inlet, the interface inlet comprising a set of electrical contacts, and
the first set of wires (46) of the nasal cannula interface (17) provides electrical communication between the one or more sensors (29) and the set of electrical contacts of the interface inlet; and
wherein the electrical contacts of the gases inlet conduit (62) and electrical contacts of the inspiratory conduit (16) are configured such that an electrical connection is formed automatically if the gases inlet conduit (62) and the inspiratory conduit (16) are pneumatically connected.

2. The nasal cannula interface (17) of claim 1, wherein the at least one prong (33,34) is configured to be received in one or more nares of the patient; and/or wherein the at least one prong (33,34) is configured to be received in one of the nares of the patient in an unsealed manner.

3. The nasal cannula interface (17) of any of claims 1 to 2, wherein at least one of the one or more sensors (29) is a patient sensor, and the parameter is a physiological parameter of the patient; and/or wherein at least one of the one or more sensors (29) is a pulse oximeter, and wherein the pulse oximeter is a reflectance type pulse oximeter.

4. The nasal cannula interface (17) of any of claims 1 to 3, wherein the body of the nasal cannula (30) further comprises a pair of side arms (31); and/or wherein the nasal cannula interface (17) further comprises a head securement assembly; or
wherein the body of the nasal cannula (30) further comprises a pair of side arms (31) and the nasal cannula interface (17) further comprises a head securement assembly, the head securement assembly being connected to the side arms (31); and wherein the head securement assembly comprises one or more straps.

5. The nasal cannula interface (17) of any of claims 1 to 4, wherein the sensors (29) are moveable relative to the body of the nasal cannula interface (17).

6. The nasal cannula interface (17) of any of claims 1 to 5, further comprising a sensor arm (47), wherein the one or more sensors (29) are located on the sensor arm (47).

7. The nasal cannula interface (17) of claim 6, wherein the sensor arm (47) is rigid such that it cannot be easily bent by a user; or wherein the sensor arm (47) is resiliently deformable such that it can be easily bent by a user.

8. The nasal cannula interface (17) of and of claims 6 to 7, wherein a surface of the sensor arm (47) comprises an adhesive such that the surface is capable of adhering to the patient's skin.

9. The nasal cannula interface (17) of any of claims 6 to 8, wherein a length of the sensor arm (47) is adjustable; and wherein the length of sensor arm (47) is adjustable through telescopic motion.

10. The nasal cannula interface (17) of any of claims 6 to 9 when dependent on claim 4, wherein the head securement assembly further comprises a sensor mount (48) connected to one of the straps, wherein the sensor arm (47) projects from the sensor mount (48).

11. The nasal cannula interface (17) of claim 10, wherein the sensor mount (48) is moveably connected to one of the straps; and wherein the sensor mount (48) is slidably connected to one of the straps.

12. The nasal cannula interface (17) of any of claims 10 to 11, wherein the sensor mount (48) is removably connected to one of the straps.

13. The nasal cannula interface (17) of any of claims 10 to 12 when dependent from claim 6, wherein the sensor arm (47) is moveable relative to the sensor mount (48); and wherein the sensor arm (47) is slidably mounted to the sensor mount (48).

14. The nasal cannula interface (17) of claim 13, wherein the sensor arm (47) is slidably mounted to the sensor mount (48) so as to be able to slide in a direction parallel to the length of the strap or gases inlet conduit (62) to which the sensor mount (48) is connected; and/or wherein the sensor arm (47) is slidably mounted to the sensor mount (48) so as to be able to slide in a direction transverse to the length of the strap or gases inlet conduit (62) which the sensor mount (48) is connected to.

15. The nasal cannula interface (17) of any of claims 1 to 14, wherein at least a first portion of the first set of wires (46) of the nasal cannula interface (17) are embedded into at least a portion of the body of the nasal cannula (30), the interface connector (35) of the nasal cannula (30), or the gases inlet conduit (62); and/or wherein at least a second portion of the first set of wires (46) of the nasal cannula interface (17) are located on an outer surface of at least a portion of the body of the nasal cannula (30), the interface connector (35) of the nasal cannula (35), or the gases inlet conduit (60); and/or wherein at least a third portion of the first set of wires (46) of the nasal cannula interface (17) are located on an inner surface of at least a portion of the body of the nasal cannula (30), the interface connector (35) of the nasal cannula (30), or the gases inlet conduit (62).

16. A respiratory support system for generating a gases flow comprising:
a respiratory support apparatus (10) comprising:
a flow generator (11),
a respiratory support apparatus outlet (21), and
a controller (13),
an inspiratory conduit (16) comprising:
a patient end having an inspiratory conduit outlet, and
an apparatus end having an inspiratory conduit inlet,
the nasal cannula interface (17) of any of the preceding claims;
wherein the respiratory support apparatus outlet (21) is configured to form a pneumatic and electrical connection with the inspiratory conduit inlet,
wherein the respiratory support apparatus outlet (21) is in electrical communication with the controller (13), and
wherein the controller (13) is configured to supply power to and receive data from the one or more sensors (29).

## Patentansprüche

1. Nasenkanülenschnittstelle (17) zum Bereitstellen einer Gasströmung an einen Patienten, umfassend:
eine Gaseinlassleitung (62), die dazu konfiguriert ist, die Gasströmung von einer Strömungsquelle über eine inspiratorische Leitung (16) aufzunehmen, wobei die Gaseinlassleitung (62) mindestens einen Abschnitt eines Gasströmungswegs definiert und ein Patientenende und ein distales Ende umfasst,
einen ersten Satz Drähte (46),
einen oder mehrere Sensoren (29), die dazu konfiguriert sind, auf der Haut des Patienten platziert zu werden, und dazu konfiguriert sind, mindestens einen Parameter zu messen, und
eine Nasenkanüle (30), umfassend
einen Körper (32) mit einem Basisabschnitt (39) und mindestens einem Prong (33, 34), der sich von dem Basisabschnitt (39) erstreckt, wobei der mindestens eine Prong (33, 34) dazu konfiguriert ist, die Gasströmung zu einem Atemweg des Patienten zu leiten, und
einen Schnittstellenverbinder (35) zum Aufnehmen der Gasströmung von der Gaseinlassleitung (62) und Leiten der Gasströmung zu dem mindestens einen Prong (33, 34), und wobei das Patientenende mit dem Schnittstellenverbinder (35) verbunden ist,
wobei das distale Ende einen Schnittstelleneinlass umfasst, wobei der Schnittstelleneinlass einen Satz elektrischer Kontakte umfasst, und
wobei der erste Satz Drähte (46) der Nasenkanülenschnittstelle (17) eine elektrische Kommunikation zwischen dem einen oder den mehreren Sensoren (29) und dem Satz elektrischer Kontakte des Schnittstelleneinlasses bereitstellt; und
wobei die elektrischen Kontakte der Gaseinlassleitung (62) und die elektrischen Kontakte der inspiratorischen Leitung (16) so konfiguriert sind, dass eine elektrische Verbindung automatisch gebildet wird, wenn die Gaseinlassleitung (62) und die inspiratorische Leitung (16) pneumatisch verbunden werden.

2. Nasenkanülenschnittstelle (17) nach Anspruch 1, wobei die mindestens ein Prong (33, 34) dazu konfiguriert ist, in einem oder mehreren Nasenlöchern des Patienten aufgenommen zu werden; und/oder wobei der mindestens eine Prong (33, 34) dazu konfiguriert ist, in einem der Nasenlöcher des Patienten in einer nicht abgedichteten Weise aufgenommen zu werden.

3. Nasenkanülenschnittstelle (17) nach einem der Ansprüche 1 bis 2, wobei mindestens einer des einen oder der mehreren Sensoren (29) ein Patientensensor ist und der Parameter ein physiologischer Parameter des Patienten ist; und/oder wobei mindestens einer des einen oder der mehreren Sensoren (29) ein Pulsoximeter ist, und wobei das Pulsoximeter ein Pulsoximeter vom Reflexionstyp ist.

4. Nasenkanülenschnittstelle (17) nach einem der Ansprüche 1 bis 3, wobei der Körper der Nasenkanüle (30) ferner ein Paar Seitenarme (31) umfasst; und/oder wobei die Nasenkanülenschnittstelle (17) ferner eine Kopfsicherungsanordnung umfasst; oder
wobei der Körper der Nasenkanüle (30) ferner ein Paar Seitenarme (31) umfasst und die Nasenkanülenschnittstelle (17) ferner eine Kopfsicherungsanordnung umfasst, wobei die Kopfsicherungsanordnung mit den Seitenarmen (31) verbunden ist; und wobei die Kopfsicherungsanordnung einen oder mehrere Riemen umfasst.

5. Nasenkanülenschnittstelle (17) nach einem der Ansprüche 1 bis 4, wobei die Sensoren (29) relativ zu dem Körper der Nasenkanülenschnittstelle (17) beweglich sind.

6. Nasenkanülenschnittstelle (17) nach einem der Ansprüche 1 bis 5, ferner umfassend einen Sensorarm (47), wobei der eine oder die mehreren Sensoren (29) an dem Sensorarm (47) angeordnet sind.

7. Nasenkanülenschnittstelle (17) nach Anspruch 6, wobei der Sensorarm (47) starr ist, so dass er von einem Benutzer nicht einfach gebogen werden kann; oder wobei der Sensorarm (47) resilient verformbar ist, so dass er von einem Benutzer einfach gebogen werden kann.

8. Nasenkanülenschnittstelle (17) nach einem der Ansprüche 6 bis 7, wobei eine Oberfläche des Sensorarms (47) einen Haftstoff aufweist, so dass die Oberfläche in der Lage ist, an der Haut des Patienten zu haften.

9. Nasenkanülenschnittstelle (17) nach einem der Ansprüche 6 bis 8, wobei eine Länge des Sensorarms (47) einstellbar ist; und wobei die Länge des Sensorarms (47) durch eine Teleskopbewegung einstellbar ist.

10. Nasenkanülenschnittstelle (17) nach einem der Ansprüche 6 bis 9, wenn abhängig von Anspruch 4, wobei die Kopfsicherungsanordnung ferner eine Sensorhalterung (48) umfasst, die mit einem der Riemen verbunden ist, wobei der Sensorarm (47) von der Sensorhalterung (48) vorsteht.

11. Nasenkanülenschnittstelle (17) nach Anspruch 10, wobei die Sensorhalterung (48) beweglich mit einem der Riemen verbunden ist; und wobei die Sensorhalterung (48) verschiebbar mit einem der Riemen verbunden ist.

12. Nasenkanülenschnittstelle (17) nach einem der Ansprüche 10 bis 11, wobei die Sensorhalterung (48) entfernbar mit einem der Riemen verbunden ist.

13. Nasenkanülenschnittstelle (17) nach einem der Ansprüche 10 bis 12, wenn abhängig von Anspruch 6, wobei der Sensorarm (47) relativ zu der Sensorhalterung (48) beweglich ist; und wobei der Sensorarm (47) verschiebbar an der Sensorhalterung (48) angebracht ist.

14. Nasenkanülenschnittstelle (17) nach Anspruch 13, wobei der Sensorarm (47) verschiebbar an der Sensorhalterung (48) angebracht ist, so dass dieser in einer Richtung parallel zu der Länge des Riemens oder der Gaseinlassleitung (62), mit der die Sensorhalterung (48) verbunden ist, verschiebbar ist; und/oder wobei der Sensorarm (47) verschiebbar an der Sensorhalterung (48) angebracht ist, so dass dieser in einer Richtung quer zu der Länge des Riemens oder der Gaseinlassleitung (62), mit der die Sensorhalterung (48) verbunden ist, verschiebbar ist.

15. Nasenkanülenschnittstelle (17) nach einem der Ansprüche 1 bis 14, wobei mindestens ein erster Abschnitt des ersten Satzes Drähte (46) der Nasenkanülenschnittstelle (17) in mindestens einen Abschnitt des Körpers der Nasenkanüle (30), den Schnittstellenverbinder (35) der Nasenkanüle (30) oder die Gaseinlassleitung (62) eingebettet ist; und/oder wobei mindestens ein zweiter Abschnitt des ersten Satzes Drähte (46) der Nasenkanülenschnittstelle (17) an einer Außenfläche von mindestens einem Abschnitt des Körpers der Nasenkanüle (30), dem Schnittstellenverbinder (35) der Nasenkanüle (35) oder der Gaseinlassleitung (60) angeordnet ist; und/oder wobei mindestens ein dritter Abschnitt des ersten Satzes Drähte (46) der Nasenkanülenschnittstelle (17) an einer Innenfläche von mindestens einem Abschnitt des Körpers der Nasenkanüle (30), dem Schnittstellenverbinder (35) der Nasenkanüle (30) oder der Gaseinlassleitung (62) angeordnet ist.

16. Atemunterstützungssystem zum Erzeugen einer Gasströmung, umfassend:
eine Atemunterstützungsvorrichtung (10), umfassend:
einen Strömungsgenerator (11),
einen Atemunterstützungsvorrichtungsauslass (21), und
eine Steuerung (13),
eine inspiratorische Leitung (16), umfassend:
ein Patientenende mit einem inspiratorischen Leitungsauslass, und
ein Vorrichtungsende mit einem inspiratorischen Leitungseinlass,
die Nasenkanülenschnittstelle (17) nach einem der vorhergehenden Ansprüche;
wobei der Atemunterstützungsvorrichtungsauslass (21) dazu konfiguriert ist, eine pneumatische und elektrische Verbindung mit dem inspiratorischen Leitungseinlass zu bilden,
wobei der Atemunterstützungsvorrichtungsauslass (21) in elektrischer Kommunikation mit der Steuerung (13) steht, und
wobei die Steuerung (13) dazu konfiguriert ist, den einen oder die mehreren Sensoren (29) mit Leistung zu versorgen und Daten von diesen zu empfangen.

## Revendications

1. Interface de canule nasale (17) destinée à fournir un flux de gaz à un patient, comprenant :
un conduit d'entrée de gaz (62) configuré pour recevoir le flux de gaz d'une source de flux via un conduit inspiratoire (16), le conduit d'entrée de gaz (62) définissant au moins une partie d'un trajet de flux de gaz et comprenant une extrémité patient et une extrémité distale,
un premier ensemble de fils (46),
un ou plusieurs capteurs (29) configurés pour être placés sur la peau du patient et configurés pour mesurer au moins un paramètre, et
une canule nasale (30) comprenant
un corps (32) ayant une partie de base (39) et au moins une pointe (33, 34) s'étendant à partir de la partie de base (39), l'au moins une pointe (33, 34) étant configurée pour diriger le flux de gaz vers une voie respiratoire du patient, et
un connecteur d'interface (35) destiné à recevoir le flux de gaz provenant du conduit d'entrée de gaz (62) et à diriger le flux de gaz vers l'au moins une pointe (33, 34), et l'extrémité patient étant connectée au connecteur d'interface (35),
l'extrémité distale comprenant une entrée d'interface, l'entrée d'interface comprenant un ensemble de contacts électriques, et
le premier ensemble de fils (46) de l'interface de canule nasale (17) assurant la communication électrique entre le ou les capteurs (29) et l'ensemble de contacts électriques de l'entrée d'interface ; et
les contacts électriques du conduit d'entrée de gaz (62) et les contacts électriques du conduit inspiratoire (16) étant configurés de telle sorte qu'une connexion électrique est formée automatiquement si le conduit d'entrée de gaz (62) et le conduit inspiratoire (16) sont connectés pneumatiquement.

2. Interface de canule nasale (17) selon la revendication 1, l'au moins une pointe (33, 34) étant configurée pour être reçue dans une ou plusieurs narines du patient ; et/ou l'au moins une pointe (33, 34) étant configurée pour être reçue dans l'une des narines du patient d'une manière non scellée.

3. Interface de canule nasale (17) selon l'une quelconque des revendications 1 et 2, au moins l'un du ou des capteurs (29) étant un capteur de patient, et le paramètre étant un paramètre physiologique du patient ; et/ou au moins l'un du ou des capteurs (29) étant un oxymètre de pouls, et l'oxymètre de pouls étant un oxymètre de pouls du type réflectance.

4. Interface de canule nasale (17) selon l'une quelconque des revendications 1 à 3, le corps de la canule nasale (30) comprenant en outre une paire de bras latéraux (31) ; et/ou l'interface de canule nasale (17) comprenant en outre un ensemble de fixation de tête ; ou le corps de la canule nasale (30) comprenant en outre une paire de bras latéraux (31) et l'interface de canule nasale (17) comprenant en outre un ensemble de fixation de tête, l'ensemble de fixation de tête étant connecté aux bras latéraux (31) ; et l'ensemble de fixation de tête comprenant une ou plusieurs sangles.

5. Interface de canule nasale (17) selon l'une quelconque des revendications 1 à 4, les capteurs (29) étant mobiles par rapport au corps de l'interface de canule nasale (17).

6. Interface de canule nasale (17) selon l'une quelconque des revendications 1 à 5, comprenant en outre un bras de capteur (47), le ou les capteurs (29) étant situés sur le bras de capteur (47).

7. Interface de canule nasale (17) selon la revendication 6, le bras de capteur (47) étant rigide de sorte qu'il ne peut pas être facilement plié par un utilisateur ; ou le bras de capteur (47) étant élastiquement déformable de sorte qu'il peut être facilement plié par un utilisateur.

8. Interface de canule nasale (17) selon et selon les revendications 6 à 7, une surface du bras de capteur (47) comprenant un adhésif de sorte que la surface est capable d'adhérer à la peau du patient.

9. Interface de canule nasale (17) selon l'une quelconque des revendications 6 à 8, une longueur du bras de capteur (47) étant réglable ; et la longueur du bras de capteur (47) étant réglable par un mouvement télescopique.

10. Interface de canule nasale (17) selon l'une quelconque des revendications 6 à 9 lorsqu'elles dépendent de la revendication 4, l'ensemble de fixation de tête comprenant en outre une monture de capteur (48) connectée à l'une des sangles, le bras de capteur (47) faisant saillie à partir de la monture de capteur (48).

11. Interface de canule nasale (17) selon la revendication 10, la monture de capteur (48) étant connectée de manière mobile à l'une des sangles ; et la monture de capteur (48) étant connectée de manière coulissante à l'une des sangles.

12. Interface de canule nasale (17) selon l'une quelconque des revendications 10 et 11, la monture de capteur (48) étant connectée de manière amovible à l'une des sangles.

13. Interface de canule nasale (17) selon l'une quelconque des revendications 10 à 12 lorsqu'elles dépendent de la revendication 6, le bras de capteur (47) étant mobile par rapport à la monture de capteur (48) ; et le bras de capteur (47) étant monté de manière coulissante sur la monture de capteur (48).

14. Interface de canule nasale (17) selon la revendication 13, le bras de capteur (47) étant monté de manière coulissante sur la monture de capteur (48) de manière à pouvoir coulisser dans une direction parallèle à la longueur de la sangle ou du conduit d'entrée de gaz (62) auquel la monture de capteur (48) est connectée ; et/ou le bras de capteur (47) étant monté de manière coulissante sur la monture de capteur (48) de manière à pouvoir coulisser dans une direction transversale à la longueur de la bande ou du conduit d'entrée de gaz (62) auquel la monture de capteur (48) est connectée.

15. Interface de canule nasale (17) selon l'une quelconque des revendications 1 à 14, au moins une première partie du premier ensemble de fils (46) de l'interface de canule nasale (17) étant intégrée dans au moins une partie du corps de la canule nasale (30), le connecteur d'interface (35) de la canule nasale (30), ou le conduit d'entrée de gaz (62) ; et/ou au moins une deuxième partie du premier ensemble de fils (46) de l'interface de canule nasale (17) étant située sur une surface extérieure d'au moins une partie du corps de la canule nasale (30), du connecteur d'interface (35) de la canule nasale (35) ou du conduit d'entrée de gaz (60) ; et/ou au moins une troisième partie du premier ensemble de fils (46) de l'interface de canule nasale (17) étant située sur une surface interne d'au moins une partie du corps de la canule nasale (30), du connecteur d'interface (35) de la canule nasale (30) ou du conduit d'entrée de gaz (62).

16. Système d'assistance respiratoire pour générer un flux de gaz comprenant :
un appareil d'assistance respiratoire (10) comprenant :
un générateur de flux (11),
une sortie d'appareil d'assistance respiratoire (21), et
un dispositif de commande (13),
un conduit inspiratoire (16) comprenant :
une extrémité patient ayant une sortie de conduit inspiratoire, et
une extrémité d'appareil ayant une entrée de conduit inspiratoire,
l'interface de canule nasale (17) selon l'une quelconque des revendications précédentes ;
la sortie de l'appareil d'assistance respiratoire (21) étant configurée pour former une connexion pneumatique et électrique avec l'entrée du conduit inspiratoire,
la sortie (21) de l'appareil d'assistance respiratoire étant en communication électrique avec le dispositif de commande (13), et
le dispositif de commande (13) étant configuré pour alimenter en énergie et recevoir des données du ou des capteurs (29).
